(19) Europäisches Patentamt
European Patent Office
Office européen des brevets

(11) EP 3 115 196 B1

(12) EUROPEAN PATENT SPECIFICATION

(45) Date of publication and mention of the grant of the patent:
**09.10.2019 Bulletin 2019/41**

(21) Application number: **15871307.3**

(22) Date of filing: **13.11.2015**

(51) Int Cl.:
***B32B 7/14*** *(2006.01)* ***B32B 5/26*** *(2006.01)*
***B32B 29/02*** *(2006.01)*

(86) International application number:
**PCT/JP2015/082637**

(87) International publication number:
**WO 2016/181579 (17.11.2016 Gazette 2016/46)**

# (54) CLOTH FOR DISPOSABLE TEXTILE PRODUCT, AND DISPOSABLE TEXTILE PRODUCT USING SAME

GRUNDGEWEBE FÜR WEGWERFBARES TEXTILPRODUKT UND WEGWERFBARES TEXTILPRODUKT DAMIT

TISSU POUR PRODUIT TEXTILE JETABLE ET PRODUIT TEXTILE JETABLE UTILISANT CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2015 JP 2015097780**
**18.06.2015 JP 2015123315**
**07.08.2015 JP 2015157776**
**17.08.2015 US 201562205936 P**

(43) Date of publication of application:
**11.01.2017 Bulletin 2017/02**

(73) Proprietor: **Yamada, Kikuo**
**Shinagawa-ku**
**Tokyo 141-0022 (JP)**

(72) Inventor: **Yamada, Kikuo**
**Shinagawa-ku**
**Tokyo 141-0022 (JP)**

(74) Representative: **Ishiguro, Masaoki**
**IPecunia Patents B.V.**
**P.O. Box 593**
**6160 AN Geleen (NL)**

(56) References cited:
**EP-A1- 0 604 731**
**WO-A1-2015/046401**
**WO-A2-2014/196669**
**JP-A- H08 108 504**
**JP-A- 2006 027 089**
**JP-A- 2008 525 672**
**JP-A- 2009 297 096**
**JP-U- 3 099 698**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 115 196 B1

## Description

[Technical Field]

The present invention relates to a base fabric for a disposable textile product which can be used for the manufacture of the disposable textile product, such as a disposable undergarment and diaper. The present invention also relates to the disposable textile product manufactured using the base fabric.

[Background Art]

A base fabric for a disposable textile product has been widely used for diaper, hygiene product, and also undergarment, clothing, and bedding that can be used in a makeshift or temporary manner for travel, for an emergency situation, outdoor, in a hospital, facility, and hotel. The base fabric is required to ensure good feeling in wearing when the base fabric product is worn on the body. For example, in the case of diaper, hygiene product, or undergarment, important property which is required for the base fabric includes good body-fit feeling, good contact feeling when in contact with the skin, good air permeability, and good moisture transpiration ability which results in sweat absorption and drying ability (referred to hereinbelow simply as moisture transpiration ability).
In the case of a diaper which is one disposable fabric product, nonwoven fabric material is used most often as the material base fabric with consideration for a function thereof. For example, PTL1 discloses a disposable diaper in which an inner pad is attached to a diaper body, wherein the diaper body is configured by inserting an absorbent such as cotton pulp between a liquid-permeable top sheet made of nonwoven fabric and a liquid-impermeable back sheet made of polyethylene, etc. , and the inner pad is likewise configured by inserting an absorbent such as cotton pulp between a liquid-permeable top sheet made of nonwoven fabric and a liquid-impermeable back sheet made of polyethylene, etc.
In the case of disposable sleeping garment provided to a guest in a hotel, and the like, it has been suggested to impart a skin care function thereto. For example, PTL2 discloses a disposable sleeping garment that is subjected to processing of impregnating or coating a nonwoven base fabric with a lipophilic moisturizer, this processing constituting the skin care function processing.

[Citation List]

[Patent Literature]

[PTL 1] Japanese Patent No. 3667267
[PTL 2] Japanese Patent Application Publication No. 2009-97104

Other base fabric and disposable textile product are described in WO2015/046401, EP0604731, JP2009297096, WO2014/196669.

[Summary of Invention]

[Technical Problem]

In PTL1, there is a problem in that although the nonwoven fabric which is used as a material for the liquid-permeable top sheet has air permeability, the moisture transpiration ability thereof is low. Further, in the diaper disclosed in PTL1, although a liquid-impermeable back sheet is used for preventing bodily wastes such as urine from leaking to the outside, this liquid-impermeable back sheet, too, has inherently low moisture transpiration ability. It is generally known that the back sheet in the diaper is also imparted with moisture permeability, but no contribution is made thereby to the improvement of the moisture transpiration ability. Thus, the base fabric material for use in the conventional disposable diaper does not have sufficient moisture transpiration ability, and water vapor formed by sweat, or the like, stays inside the diaper, thereby inevitably causing uncomfortable feeling of sweatiness and stickiness.
Another drawback of the base fabric material used in the conventional disposable diaper is that they have poor heat dissipation ability, which is the ability to dissipate the heat generated from the body to the outside, and the heat accumulated in the diaper creates a discomfort. Also, when wearing the diaper, contact with the fiber material creates an unpleasant skin sensation peculiar to a fiber material, lacking the pleasant cool contact feeling and smoothness such as those inherent to a cotton material, and the refreshing feeling of wearing it is absent. Thus, there is room for improvement in the field of base fabric configuring disposable a textile product.

The drawback of the disposable sleeping garment disclosed in PTL2 is that the base fabric itself does not have a skin care function, and the intended skin care function is imparted by post-processing such as impregnation or coating with the lipophilic moisturizer, and the manufacturing process is complex and costly.

As for the base fabric material for disposable textile product that is in direct contact with the human skin, the base fabric itself is generally required to be flexible and create an overall soft and plump feeling. Further, from the standpoint of appearance and visibility, the base fabric for clothing for traveling, an emergency situation, and outdoor is required to be capable of representing efficiently and effectively a variety of highly aesthetic design, and is also required to include media property such as messaging, promotion, and advertising. In addition, it is also desirable that various function could be imparted thereto according to application.

It is an object of the present invention to provide a base fabric for the disposable textile product that excels in moisture transpiration ability, heat dissipation ability, and moisture permeability. It is another object of the present invention to provide the disposable textile product that creates excellent feeling in wearing and excels in contact with the skin, moisture transpiration ability, heat dissipation ability, and moisture permeability by using the base fabric in accordance with the present invention as the base fabric for the disposable textile product.

Yet another object of the present invention is to provide the base fabric for the disposable textile product that can efficiently and effectively express a variety of design and message and excels in overall soft and plump feeling, flexibility, and comfort in use while utilizing the texture that is characteristic to the base fabric material itself. Still another object of the present invention is to provide the base fabric for the disposable textile product that makes it possible to impart efficiently and effectively a variety of functions thereto according to the application and is easy to manufacture, and also to provide the disposable textile product utilizing the property of the base fabric.

[Solution to Problem]

The base fabric for a disposable textile product is configured in accordance with claims 1-11.

An elastic member is provided, as means for imparting the elasticity to the laminated sheet, between the fiber material and the second fibrous sheet.

The first fibrous sheet and the fiber material are preferably joined together with a hot-melt adhesive through the elastic member. The second fibrous sheet and the fiber material are preferably joined together by the hot-melt adhesive.

The elastic member is configured of a plurality of linear elastic body having stretching ability, and the plurality of linear elastic body is disposed at an interval in the width direction of the laminated sheet and joined between the first fibrous sheet and the fiber material.

The linear elastic body may be provided over the entire region or in a partial region inside the laminated sheet.

The base fabric in accordance with the present invention has a configuration in which, in the laminated sheet, a plurality of a shirring portion extending in a direction perpendicular to the longitudinal direction of the linear elastic body in a non-tensioned state is formed, and a shirring row is formed in a pattern.

The fiber material preferably has a flexible structure obtained by a mechanical softening process. It is also preferred that the fiber material be subjected to a weakening process.

It is preferred that the first fibrous sheet and the second fibrous sheet be configured of a material having the air permeability and the moisture permeability, and nonwoven fabric is preferably used as such a material.

It is preferred that the fiber material be configured of a material having the liquid diffusibility and liquid permeability.

The disposable textile product in accordance with claims 12-14 is configured using the base fabric for the disposable product which is disclosed hereinabove.

The disposable textile product in accordance with the present invention can be configured as an undergarment, diaper, fitness wear, swimming wear, tube top, room wear, raincoat, or belly band.

[Advantageous Effect of Invention]

The base fabric in accordance with the present invention has excellent moisture transpiration ability, heat dissipation ability, and moisture permeability, and when the base fabric of the present invention is used for the disposable textile product, good feeling in wearing can be imparted to the product.

The base fabric in accordance with the present invention also has the elasticity and therefore excels in flexibility and contact with the skin. Furthermore, the elasticity enables the production of a free-size product.

The disposable textile product of the present invention is configured using the above-described base fabric, has refreshing feeling in wearing, creates soft feeling in contact with the skin, and demonstrates excellent moisture transpiration ability, heat dissipation ability, and moisture permeability.

[Brief Description of Drawings]

Fig. 1 is a plan view illustrating an embodiment of the base fabric for a disposable textile product of the present invention.
Fig. 2 is a perspective view of the base fabric depicted in Fig. 1.
Fig. 3 is a principal enlarged view of the base fabric depicted in Fig. 1.
Fig. 4 is an end surface view in the cross section taken along the A-A line in Fig. 3.
Fig. 5 is an end surface view in the cross section taken along the B-B line in Fig. 3.
Figs. 6a to 6j are simplified drawings illustrating the arrangement of an elastic member constituting the base fabric of the present invention.
Fig. 7 is a front view of an undergarment (T-shirt) which is the disposable textile product in accordance with the present invention, this product being configured by using the base fabric of the present invention.
Fig. 8 is a perspective view illustrating the wearing state of a garment (tube top) which is the disposable textile product in accordance with the present invention.
Fig. 9 is a front view of a belly band which is the disposable textile product in accordance with the present invention.
Fig. 10 is a perspective view of a rainwear which is the disposable textile product in accordance with the present invention.
Figs. 11a to 11e are perspective views illustrating the wearing state of a supporter which is the disposable textile product in accordance with the present invention.
Fig. 12 is a perspective view of a bandage which is the disposable textile product in accordance with the present invention.
Figs. 13a to 13c are explanatory drawings illustrating the action of the shirring portion in the base fabric of the present invention.
Fig. 14 is a principal cross-sectional view of the laminated sheet constituting the base fabric of the present invention.
Fig. 15 is a conceptual diagram illustrating the space formed between the second fibrous sheet and fiber material.
Fig. 16 is a simplified drawing illustrating the formation of an adhesive permeation portion in the layer of the fiber material.
Fig. 17 illustrating the wearing state of a diaper of a pants type which the disposable textile product in accordance with the present invention.
Fig. 18 is an explanatory drawing illustrating a moisture transpiration ability (II) test (Boken standard BQEA028) in an example.
Fig. 19 illustrates the transpiration ratio representing the moisture transpiration ability in the example and a comparative example.
Fig. 20 illustrates the moisture permeability representing the moisture permeability in the example and comparative example.

[Description of Embodiment]

An embodiment of the present invention will be explained hereinbelow with reference to the appended drawing. Fig. 1 illustrates an embodiment representing the surface state of a base fabric 1 of the embodiment of the present invention. In Fig. 1, the base fabric 1 is continuous in the x direction which is the first direction. The reference numeral 5 denotes an elastic member provided inside the base fabric. The figure illustrates a configuration in which a large number of uneven surface is formed by a large number of the elastic member 5. As depicted in Fig. 2, a large number of shirring portion 6 is formed by the large number of uneven surface.
As depicted in Figs. 4 and 5, the base fabric 1 is configured of a laminated sheet 30 which has a first fibrous sheet 2 having air permeability, a second fibrous sheet 3 likewise having the air permeability, and a fiber material 4 which is interposed between the first fibrous sheet 2 and the second fibrous sheet 3 and has liquid diffusibility, the laminated sheet being obtained by laminating the first fibrous sheet 2 and the second fibrous sheet 3 and the fiber material 4. The first fibrous sheet 2 and the second fibrous sheet 3 constitute a fiber layer having the air permeability, and the fiber material 4 constitutes the fiber layer having the liquid diffusibility. Thus, the laminated sheet 30 forms a composite layer 31 in which the fiber layer having the air permeability (air-permeable fiber layer) and the fiber layer having the liquid diffusibility (liquid-diffusible fiber layer) are laminated. The composite layer 31 has a three-layer structure including a first air-permeable fiber layer, the liquid diffusible fiber layer adjacent to the first air-permeable fiber layer, and a second air-permeable fiber layer, adjacent to the liquid diffusible fiber layer.
The first fibrous sheet 2 and the fiber material 4 are intermittently joined together through the elastic member 5. The second fibrous sheet 3 and the fiber material 4 are intermittently joined together. Adhesive bonding, thermal fusion, and ultrasonic welding can be used as joining means, but from the standpoint of easiness of operation, adhesive bonding is preferred. The embodiment of the present invention will be explained hereinbelow with reference to the case in which the adhesive bonding is used as the joining means.

In adhesive bonding as the joining means, the hot-melt adhesive is preferably used as the adhesive. The embodiment of the present invention will be explained hereinbelow with reference to the case in which the hot-melt adhesive is used as the adhesive. In order to join the second fibrous sheet 3 and the fiber material 4 intermittently together by using the hot-melt adhesive, as depicted in Fig. 14, the hot-melt adhesive is intermittently applied to the fiber material 4 (alternatively, the hot-melt adhesive may be intermittently applied to the second fibrous sheet 3), and the second fibrous sheet and the fiber material are laminated and integrated by joining. In this case, a non-adhesive portion 8 where an adhesive layer 7 is not present is formed between the second fibrous sheet 3 and the fiber material 4, and a space 9 is formed by such non-adhesive portion 8. Since the elastic member 5 is interposed between the first fibrous sheet 2 and the fiber material 4, the intermittent joining of the first fibrous sheet 2 and the fiber material 4 is performed by joining the first fibrous sheet 2 and the fiber material 4 together in the location of the elastic member 5. Thus, as depicted in Fig. 14, the hot-melt adhesive is applied by spraying to the outer circumference of the large number of the elastic member 5 arranged parallel to each other at a predetermined interval, the elastic member 5 to which the hot-melt adhesive has been applied is positioned between the first fibrous sheet 2 and the surface of the fiber material 4 in the laminated sheet of the second fibrous sheet 3 and the fiber material 4, lamination is performed such that the elastic member 5 is inserted between the surface of the fiber material 4 in the laminated sheet and the first fibrous sheet 2, and the layers are integrated by joining. The opposing surface of the first fibrous sheet 2 and the fiber material 4 where the elastic member 5 is not present becomes the non-adhesive portion 8, and the space 9 is formed by the non-adhesive portion 8 (this feature is not specifically illustrated by Fig. 14) . Fig. 15 illustrates the formation of the space 9 by the non-adhesive portion 8 in the shirring portion 6. Such a structure in which the space 9 is formed by the non-adhesive portion 8 improves a function such as moisture transpiration ability, heat dissipation ability, and the moisture permeability in the base fabric 1.

As depicted in Fig. 4, the uneven surface is formed by the composite layer 31 in the laminated sheet 30, and the shirring portion 6 is formed by the uneven surface. As depicted in Figs. 2, 3, and 4, the shirring portion 6 is formed by a protrusion 6a and a depression 6b in the uneven surface, and a large number of the shirring portion 6 is configured by continuous formation of the protrusion 6a and depression 6b.

Arranging the elastic member 5 inside the base fabric 1 in the above-described manner is means for forming the shirring portion 6 constituted by the protrusion 6a and the depression 6b. A linear elastic body 5a having stretching ability is used as the elastic member 5, and a rubber filament can be advantageously used as the linear elastic body 5a. The embodiment of the present invention will be explained hereinbelow with reference to the case in which the linear elastic body 5a is used as the elastic member 5.

As depicted in Figs. 1 and 2, the linear elastic body 5a is arranged such that the extension direction of a line thereof is the same as the longitudinal direction of the laminated sheet 30 (x direction in Figs. 1 and 2), and a large number of the linear elastic body 5a is arranged parallel to each other at a predetermined interval. Thus, a large number of the linear elastic body 5a is arranged at an interval in the width direction of the laminated sheet 30 (y direction in Figs. 1 and 2), and a large number of linear elastic body row is thus formed.

As depicted in Fig. 5, the linear elastic body 5a is disposed between the first fibrous sheet 2 and the fiber material 4. In the present invention, the number of the linear elastic body 5a per unit surface area can be set as appropriate, and the interval between the linear elastic body 5a in the linear elastic body row can be also set as appropriate. In such a configuration, one shirring portion 6 extending from one end to the other end along the y direction (second direction which is different from the first direction) in Figs . 1 and 2 will be referred to as "a shirring portion of one row". The shirring portion 6 is formed in a large number of row at a predetermined interval in the x direction in Figs. 1 and 2. The joining region of the composite layer 31 and the linear elastic body 5a in the shirring portion 6 will be referred to as a shirring portion support point. As indicted hereinabove, the number of the linear elastic body 5a per unit surface area can be set as appropriate, but where the number of the linear elastic body 5a is increased and the interval between the linear elastic body 5a is decreased, the number of shirring portion support point in the shirring portion of one row is increased. Therefore, the protrusion 6a and the depression 6b in the shirring portion 6 of one row can be formed to have a uniform shape and this shape can be maintained. Such a configuration is preferred because the shape of the shirring portion 6 is prevented from collapsing, and the flexibility, moisture transpiration ability, heat dissipation ability, and the moisture permeability of the base fabric 1 are improved. From this standpoint, it is preferred that the interval between the shirring portion 6, that is, the pitch interval between the protrusion 6a, be 2 mm to 7 mm. It is more preferred that the pitch interval between the protrusion 6a be 3.00 mm to 6.25 mm. By reducing the pitch interval between the protrusion 6a, it is possible to form fine shirring, thereby improving the external appearance. Further, since an area of contact with the skin per one shirring decreases, contact with the skin is improved, and since the surface area increases, the ability to absorb sweat, or the like, is enhanced. Meanwhile, by increasing the pitch interval between the protrusion 6a, it is possible to suppress suitably the elastic force of the rubber filament and reduce the production cost.

An example of a method for manufacturing the base fabric 1 of the present invention by using the rubber filament as the linear elastic body 5a and the hot-melt adhesive as the adhesive will be explained hereinbelow. As will be described below, a paper material is used as the fiber material 4, and nonwoven fabrics are used as the first fibrous sheet 2 and the second fibrous sheet 3. Therefore, the method for manufacturing the base fabric 1 of the present invention will be explained hereinbelow wherein the paper material is used as the fiber material 4 and the nonwoven fabric is used as the first fibrous sheet 2 and the second fibrous sheet 3. The nonwoven fabric used as the first fibrous sheet 2 is referred to as the first nonwoven fabric, and the nonwoven fabric used as the second fibrous sheet 3 is referred to as the second nonwoven fabric.

The paper material serving as the fiber material 4 is unwound from a roll wound body. A printed layer 4a (see, Figs. 4 and 14) has been formed in advance on the paper material. The unwound paper material is conveyed to an embossing roll and pushed through between the roll, thereby performing the mechanical softening process. The mechanical softening process may be also an embossing process using a flat roll, or an embossing process using a meshing roll having a large number of projection on the roll surface. In the case of the latter embossing process, a large number of fine hole is opened in the paper material. The hot-melt adhesive is intermittently blown on the paper material after the embossing process. A mechanical process other than the embossing process may be also used. Meanwhile, the second nonwoven fabric sheet serving as the second fibrous sheet 3 is unwound from the roll wound body, the unwound second nonwoven fabric sheet and the paper material to which the hot-melt adhesive has been applied are press bonded by using the flat roll, the two are laminated, and a joined body sheet is manufactured. In the joined body sheet, the second nonwoven fabric sheet and the paper material are intermittently joined together.

A large number of rubber filament is unwound from the roll wound body onto which a large number of rubber filament has been wound in parallel row. The rubber filament is unwound while being tensioned by a predetermined tension force. The hot-melt adhesive is blown on the unwound rubber filament. In this case, the adhesive is blown continuously over the entire length in the longitudinal direction of the rubber filament. The adhesive is also applied to the entire circumferential surface of the rubber filament. Meanwhile, the first nonwoven fabric sheet serving as the first fibrous sheet 2 is unwound from the roll wound body, and the unwound first nonwoven fabric sheet is fed such as to face the joined body sheet. The rubber filament is supplied such that the rubber filament to which the adhesive has been applied in the above-described manner is inserted between the joined body sheet and the first nonwoven fabric sheet. In this case, the rubber filament is supplied between the surface of the paper material of the joined body sheet and the first nonwoven fabric sheet.

The rubber filament passes between the flat roll in a state of being inserted between the surface of the paper material of the joined body sheet and the first nonwoven fabric sheet. The joined body sheet, rubber filament, and first nonwoven fabric sheet are press bonded, laminated, and integrated by the flat roll. Since the first nonwoven fabric sheet and the paper material are joined such that the rubber filament is interposed therebetween, the two are joined intermittently. The laminated sheet 30 is thus manufactured in which the second nonwoven fabric sheet and the paper material are joined together intermittently, and the first nonwoven fabric sheet and the paper material are joined together intermittently. If necessary, the laminated sheet 30 is supplied to the embossing roll and the mechanical softening process is performed. By performing such a process, it is possible to improve further the flexibility of the base fabric 1.

Since the manufactured laminated sheet 30 has a large length, the sheet is cut to obtain a predetermined length in the longitudinal direction (x direction in Figs. 1 and 2) of the laminated sheet 30. In such cutting, the first nonwoven fabric sheet, second nonwoven fabric sheet, paper material, and rubber filament are cut. As a result of the cutting, the tension force applied to the rubber filament in the tensioned state is released and the filament is contracted by the restoring force. As a result of contraction stresses appearing in this case, the force acts in the length reduction direction on the composite layer 31 constituted by the first nonwoven fabric sheet, second nonwoven fabric sheet, and paper material. Therefore, the uneven surface is formed in the composite layer 31, thereby forming the shirring portion 6. The base fabric 1 having a large number of shirring portion 6 is thus manufactured.

The configuration of the base fabric 1 of the present invention is explained hereinbelow. Under the effect of the restoring force, the linear elastic body 5a is in the contracted state, that is, non-tensioned state, a large number of the shirring portion 6 extending in the direction (the width direction of the laminated sheet 30 in Figs. 1 and 2, that, is the same direction as the y direction) perpendicular to the longitudinal direction (that is, the longitudinal direction of the laminated sheet 30 in Figs. 1 and 2, that is, the same direction as the x direction) of the linear elastic body 5a in the non-tensioned state is formed, and a shirring portion row is formed in a pattern on the laminated sheet 30.

The linear elastic body 5a may be provided over the entire region inside the laminated sheet 30 or only in part thereof. The merit of providing the linear elastic body 5a over the entire region inside the laminated sheet 30 is that since uniform elasticity is imparted to the base fabric 1, when the base fabric 1 is used as a cover sheet for disposable pants or paper diaper, it can be reliably held on the body and prevented from slipping down.

The elasticity is imparted to the laminated sheet 30 by the linear elastic body 5a arranged inside the laminated sheet 30. Therefore, where the base fabric 1 constituted by the laminated sheet 30 is pulled by hand in the x direction in Figs. 1 and 2, the linear elastic body 5a extends and the base fabric 1 is therefore also extended and expanded.

Where the hand is released from this state, the linear elastic body 5a is contracted by the restoring force, thereby restoring the original dimensional state of the base fabric 1. Since the base fabric 1 is thus stretchable, when the base fabric 1 is used for a disposable textile product such as the undergarment and diaper, the base fabric excels in fit feeling with respect to the body and, moreover, makes it possible to produce a free-size product.

The base fabric 1 can be also configured by laminating two or more laminated sheet 30. Further, the base fabric can be also configured by laminating one or two or more of the laminated sheet 30 with one or two or more other sheet.

The base fabric 1 of the present invention can be advantageously used as a material for the disposable textile product and it is useful as a material having excellent property that could not be found in the related art. The disposable textile product using the base fabric 1 of the present invention is not limited to a product which is disposed of after a single use and is also inclusive of a product that is used repeatedly in a short period of time according to a usage application and objective. The product that is repeatedly used can withstand several cycle of washing.

The first fibrous sheet 2 and the second fibrous sheet 3 are constituted by a material having the air permeability and the moisture permeability, and the nonwoven fabric is used, as mentioned hereinabove, as the fiber material having such the air permeability and the moisture permeability.

Well-known nonwoven fabric which is generally been used conventionally can be used as the nonwoven fabric in the present invention. Thus, the well-known nonwoven fabric having a fiber structure in which the fiber is oriented unidirectionally or randomly and joined by entanglement, fusion, or adhesive bonding can be used. An Example of a fiber material for the nonwoven fabric includes a natural fiber such as vegetable fiber (cellulose polymer) and animal fiber (protein polymer); a purified fiber such as Lyocell and Tencel; a regenerated fiber such as rayon and viscose rayon; a semi-synthetic fiber such as acetate; a synthetic fiber such as nylon and acrylic fiber; and a chemical fiber such as PP (polypropylene), PE (polyethylene), and PET (polyethylene terephthalate). Those fibers may be used individually or in combination of two or more thereof. If necessary, another material may be included.

The nonwoven fabric for use in the first fibrous sheet 2 and the second fibrous sheet 3 can be selected, as appropriate, according to the type or application of the product in which the base fabric 1 is to be used. For example, when the base fabric 1 is used for a garment as the disposable textile product and the first fibrous sheet 2 or the second fibrous sheet 3 comes into contact with the user's body during wearing, it is preferred that the nonwoven fabric capable of demonstrating a function such as flexibility, good feel in contact with the skin, and sweat absorption ability be used as the first fibrous sheet 2 or the second fibrous sheet 3. Meanwhile, when the first fibrous sheet 2 or the second fibrous sheet 3 is used in a state of being positioned on the front surface side of the garment which is not in contact with the user's body, it is preferred that the nonwoven fabric capable of demonstrating a function, for example, waterproofing ability, be used as the first fibrous sheet 2 or the second fibrous sheet 3.

An example of the nonwoven fabric suitable for the first fibrous sheet 2 or the second fibrous sheet 3 includes spunbonded nonwoven fabric, thermally bonded nonwoven fabric, spunlaced nonwoven fabric, dry nonwoven fabric, wet nonwoven fabric, melt-blown nonwoven fabric, chemically bonded nonwoven fabric, needle punched nonwoven fabric, stitch bonded nonwoven fabric, and steam jet nonwoven fabric. Among them, it is preferred that spunbonded nonwoven fabric, thermally bonded nonwoven fabric, and spunlaced nonwoven fabric be used.

The basis weight of the nonwoven fabric used for the first fibrous sheet 2 and the second fibrous sheet 3 is 5 $g/m^2$ to 60 $g/m^2$, more preferably 7 $g/m^2$ to 40 $g/m^2$. The reason why it is to set the basis weight of the nonwoven fabric within those ranges is that where the basis weight is less than 5 $g/m^2$, for example, when the hot-melt adhesive is applied, the hot-melt adhesive can ooze to the nonwoven fabric surface, and where the basis weight exceeds 60 $g/m^2$, a product thickness increases, production efficiency decreases, and packaging operability is degraded. When the basis weight is 7 $g/m^2$ to 40 $g/m^2$, the joining strength can be increased by using a method of joining with the hot-melt adhesive together with a joining method based on thermal sealing or ultrasonic joining.

When the base fabric 1 of the present invention is used as a material for disposable garment, as depicted in Fig. 13a, the second fibrous sheet 3 is rarely disposed on the side which is in contact with a skin 200 of the user, but when the base fabric 1 is used as a material for disposable bedding (for example, sheets), the second fibrous sheet 3 is often disposed on the side which is in contact with the skin 200 of the user. When the second fibrous sheet 3 is thus in direct contact with the human body, it is preferred that the nonwoven fabric having the function of the flexibility, good feel in contact with the skin, and sweat absorption ability be used as a material for the second fibrous sheet 3.

It is configured that the fiber material 4 be from a raw material having the liquid diffusibility and liquid permeability, and that the paper material be used, as indicated hereinabove, as such material having the liquid diffusibility and liquid permeability.

Using the paper material as the fiber material 4 is the mode for carrying out the present invention, this is is considered herein below.

The paper material formed from a material using pulp paper or pulp as a principal material can be used as the paper material. Thus, the paper material manufactured by using pulp as the raw material and employing a papermaking process can be used.

Wood pulp, synthetic pulp, waste paper pulp, etc. can be used as the raw material pulp. Further, a natural fiber such as pulp is not limiting, and a regenerated fiber such as rayon can be also used. Furthermore, the present invention can also use a toilet paper material as the raw material pulp. In this case, for example, it is possible to use the raw material pulp constituted by bleached softwood kraft pulp obtained from softwood such as red pine, Yezo spruce, Sakhalin fir, Douglas fir, hemlock, and spruce. It is also possible to use the bleached softwood kraft pulp in combination with hardwood bleached kraft pulp obtained from hardwood such as beech, oak, birch, eucalyptus, oak, poplar, and alder. From the standpoint of problem associated with the manufacturing cost and the easiness of production, it is preferred that the softwood bleached kraft pulp be used alone as the raw material pulp.

When the paper material is formed using pulp as a principal raw material, the blended pulp preferably constitutes 30% or more, more preferably 50% or more, and most preferably 80% or more. Where the pulp is blended at the abovementioned ratio, the flexibility of the base fabric 1 as a whole can be increased and production efficiency during the manufacture can be increased. Further, by increasing the pump blending ratio, it is possible to facilitate the decomposition of the waste in the soil, or the like, after the disposal. Therefore, the environmental load can be further reduced and concern for the environment can be raised. It is preferred that a paper sheet with good disintegrability in water be used as the paper material. It is also preferred that the paper material constituting the fiber material 4 have a single-layer configuration constituted by a single paper sheet, but it may also have a multilayer configuration constituted by a stack of a plurality of paper sheet. In the case of a multilayer configuration, the layer may be of the same or different material and may have the same or different thickness. The paper material constituting the fiber material 4 may be obtained by blending the pulp with nonwoven fabric. For example, a material obtained by blending the pulp with spunlaced nonwoven fabric or by blending the pulp with air-laid nonwoven fabric can be used as the paper material.

The basis weight of the paper material constituting the fiber material 4 is 7 g/m$^2$ to 50 g/m$^2$. Where the basis weight is less than 7 g/m$^2$, good feeling in wearing is difficult to achieve when wearing the disposable textile product constituted by the base fabric 1 of the present invention. Further, sufficient moisture transpiration performance and heat radiation performance is difficult to impart to the product. In addition, the strength of the base fabric 1 is insufficient. Meanwhile, where the basis weight exceeds 50 g/m$^2$, the base fabric thickness is increased, the feeling in wearing is degraded and the moisture transpiration performance and heat radiation performance are degraded. Further, since the product thickness increases, packaging of the product is made difficult. It is more preferred that the basis weight of the paper material be 10 g/m$^2$ to 30 g/m$^2$.

Colored paper can also be used as the paper material constituting the fiber material 4. With such a configuration, it is possible to provide a highly aesthetic base fabric 1.

It is preferred that the paper material constituting the fiber material 4 be imparted with the flexible structure by the mechanical softening process. The embossing process can be used as the mechanical softening process. A pair of embossing roll in which a large number of the projection is formed on the roll surface can be used in the embossing process. Where the embossing process is performed using the embossing roll, a large number of fine hole is formed in the paper material and, at the same time, the paper structure is weakened and the paper material is imparted with the flexibility. The hole may be of a round, linear, or slit-like shape. The paper material can be likewise imparted with the flexibility by providing a rough surface formed by a large number of protrusion and depression, without opening the hole in the paper material.

The embossing roll which is used in the embossing process is not limited to the roll provided with the projection, and a pair of the flat roll having the smooth roll surface without the projection may be also used. The paper structure is weakened by passing the paper material between the flat roll and applying pressure by the roll. Thus, the paper structure is weakened by a pressurizing force applied between the roll, without using a perforation process. The embossing process for imparting the paper material with the flexible structure can be also performed by combining the embossing process with the embossing roll having the projection on the roll surface with the embossing process using the flat roll.

As a result of imparting the paper material with the flexible structure in the above-described manner, the entire base fabric 1 becomes rich in the flexibility, and the feeling in wearing of the disposable textile product configured from such the base fabric 1 can be improved. Thus, the user has a sense of satisfaction created by soft feeling in wearing, without feeling discomfort due to the fact that the paper material is used as the material for the product.

A following effect results from imparting the paper material with the flexible structure. Thus, where the paper material serving as the fiber material 4 has the flexible structure, the entire laminated sheet 30 has the flexible structure and, therefore, can be easily deformed. For this reason, when the shirring portion 6 is formed by shrinkage stress of the linear elastic body 5a, the laminated sheet 30 is easily deformed, and the shirring portion 6 with a uniform shape of the protrusion 6a and the depression 6b can be formed. As a result of forming the shirring portion 6 with the uniform shape of the protrusion 6a and the depression 6b, the entire base fabric 1 increases in the flexibility, and the disposable textile product configured from the base fabric 1 can give much softer feeling in wearing to the user. Further, as a result of imparting the paper material with the flexibility, the base fabric 1 can create overall soft and plump feeling.

As will be indicated hereinbelow, the weakening process for the paper structure by the embossing process also imparts the base fabric 1 with even better moisture transpiration ability, heat dissipation ability, and the moisture permeability.

As indicated hereinabove, it is preferred that the second fibrous sheet 3 and the fiber material 4 be joined by using the hot-melt adhesive. A suitable example of the hot-melt adhesive includes an adhesive based on EVA (ethylene-vinyl acetate copolymer), PO (polyolefins), PA (polyamides), SR (silicone synthetic rubber), ACR (acryl), and PUR (polyurethane humid-air-curable), and such adhesive can be used individually or in combination of two or more thereof. In addition to the hot-melt adhesive, an organic solvent-based adhesive and a water-soluble adhesive can be used for joining the second fibrous sheet 3 and the fiber material 4.

The second fibrous sheet 3 and the fiber material 4 can be joined by applying the adhesive over a front contact surface thereof, but the above-described intermittent application is preferred. The advantage of the intermittent application over the full-surface application is that the base fabric 1 becomes flexible and the moisture transpiration ability, heat dissipation ability, and the moisture permeability of the base fabric 1 are improved. A method for the intermittent application of the adhesive can involve linear, spot-like, stripe-like, spiral, block, and pattern application, and such method can be used individually or in combination of a plurality thereof.

As a result of the intermittent application of the adhesive, the non-adhesive portion 8 is formed between the second fibrous sheet 3 and the fiber material 4. The surface area ratio of the non-adhesive portion 8 is preferably 5% to 85%, more preferably 10% to 80%, and even more preferably 30% to 75% with respect to the surface area of the second fibrous sheet 3. Where the surface area ratio of the non-adhesive portion 8 is within such range, the base fabric 1 demonstrates excellent flexibility, moisture transpiration ability, heat dissipation ability, and the moisture permeability, and also excellent overall soft and plump feeling of the base fabric 1 can be realized. As a result, an applied amount of the adhesive may be only that necessary for obtaining a predetermined bonding strength, and it is preferred that the applied amount be as small as possible.

As mentioned hereinabove, the linear elastic body 5a is disposed such that the extension direction of the filament thereof is the same as the longitudinal direction (x direction in Figs. 1 and 2) of the laminated sheet 30, a large number of the linear elastic body is arranged at an interval in the width direction (y direction in Figs. 1 and 2) of the laminated sheet 30, and a large number of linear elastic body row is formed. The parallel arrangement of the linear elastic body 5a when a large number of the linear elastic body 5a is arranged in the laminated sheet 30 is not limiting. Figs. 6a to 6j illustrate various arrangement of the linear elastic body 5a.

Thus, a possible arrangement mode of the linear elastic body 5a includes a parallel arrangement of a straight linear elastic body 5a as depicted in Fig. 6a, an arrangement in which some or all of the straight linear elastic body 5a arranged parallel to each other is provided with a cut-out section as depicted in Figs. 6b and 6c, an arrangement in which a curved and bent linear elastic body 5a is arranged parallel to each other as depicted in Figs. 6d, 6f, and 6g, and an arrangement in which a wave-like curved linear elastic body 5a is arranged side by side irregularly as depicted in Figs . 6e and 6j. The linear elastic body 5a may be also arranged in a net-like pattern as depicted in Figs. 6h and 6i. In the arrangement of the large number of the linear elastic body 5a, it is preferred that the linear elastic body 5a have the same stretching ratio, but the linear elastic body 5a having different stretching ratio may be also combined. The elastic member 5 is not limited to the linear form, and a sheet-shaped elastic body provided with a large number of hole or cut to ensure predetermined air permeability can be also used. Such sheet-shaped elastic body can be produced by using a rubber based on urethane or silicone and a rubber material such as natural rubber as a starting material. In the present invention, it is preferred that the linear elastic body 5a of a linear form be used as the elastic member 5.

The linear elastic body 5a can be joined between the first fibrous sheet 2 and the fiber material 4 by joining the linear elastic body 5a between the first fibrous sheet 2 and the fiber material 4 with an adhesive applied to the linear elastic body 5a, or the linear elastic body 5a can be joined between the first fibrous sheet 2 and the fiber material 4 with an adhesive applied to the first fibrous sheet 2 and the fiber material 4. An adhesive same as the above-described adhesive used for joining the second fibrous sheet 3 and the fiber material 4 can be used for joining the linear elastic body. It is preferred that the hot-melt adhesive be used as the adhesive.

The hot-melt adhesive is applied by spraying on the circumferential surface of the linear elastic body 5a, and the applied hot-melt adhesive acts to join the first fibrous sheet 2, the fiber material 4, and the linear elastic body 5a in a state in which the linear elastic body is inserted between the first fibrous sheet and the fiber material. Therefore, it is not necessary to apply the hot-melt adhesive to the first fibrous sheet 2 and the fiber material 4. The adhesive may be also applied to the opposing surface of the first fibrous sheet 2 and the fiber material 4 that is to be in contact with the linear elastic body 5a, instead of applying the adhesive to the linear elastic body 5a.

As indicated hereinabove, the first fibrous sheet 2, the fiber material 4, and the linear elastic body 5a are joined together in a state in which the linear elastic body is inserted between the first fibrous sheet and fiber material by using the hot-melt adhesive which has been applied by spraying to the circumferential surface of the linear elastic body 5a. However, in the joined state, part of the hot-melt adhesive penetrates into the layer of the fiber material 4, and an adhesive permeation portion Z is formed, as depicted in Fig. 16. Since the adhesive permeation portion Z is integrated with the hot-melt adhesive on the circumferential surface of the linear elastic body 5a, the adhesive permeation portion Z exhibits an anchor action in joining together the linear elastic body 5a and the fiber material 4, thereby strengthening the joining of the linear elastic body 5a and the fiber material 4 with the hot-melt adhesive. The anchor structure is thus formed by the adhesive in the fiber material 4. The resulting advantage is that a predetermined joining strength can be obtained even with a small amount of the adhesive applied to the linear elastic body 5a, the overall strength of the base fabric 1 is increased, and the base fabric 1 which is rich in fastness can be provided. In Fig. 16, the adhesive arranged between the second fibrous sheet 3 and the fiber material 4 is omitted.

A problem associated with the base fabric for the conventional disposable textile product is that when nonwoven fabric is joined together by an adhesive, the adhesive oozes to the base fabric surface through a gap between the fiber of the nonwoven fabric. By contrast, in the base fabric 1 of the present invention, since the composite layer 31 is formed and the fiber material 4 is included in the composite layer 31, the adhesive can be prevented from oozing to the base fabric surface as a result of the adhesive penetrating into the fiber material 4.

As indicated hereinabove, in the process for manufacturing the base fabric 1, the laminated sheet 30 which is manufactured in a continuous mode is cut to obtain a product of a predetermined length. Because of the large length, the cutting is performed such as to obtain the predetermined length in the longitudinal direction (x direction in Figs. 1 and 2) of the laminated sheet 30. Since the linear elastic body 5a is cut in the cutting, the linear elastic body 5a that has been under tension are released therefrom and shrunk by the restoring force. An uneven surface is formed in the composite layer 31 under the effect of shrinkage stresses created at this time. Where the joining force of the linear elastic body 5a and the first fibrous sheet 2 and the joining force of the linear elastic body 5a and the fiber material 4 are small in this case, peeling occurs between the linear elastic body 5a, the first fibrous sheet 2, and the fiber material 4, the shrinkage stress does not reach the composite layer 31 constituted by the first fibrous sheet 2 and the fiber material 4 joined together by the linear elastic body 5a, and the so-called "rubber dropout" effect occurs. As a result of the "rubber dropout", the composite layer 31 does not receive a force in the direction of reducing the length thereof, and therefore, no uneven surface is formed in the composite layer 31, resulting in non-formation of the shirring portion 6. In this case, where the adhesive permeation portion Z is formed by the hot-melt adhesive inside the layer of the fiber material 4, as depicted in Fig. 16, the joining force of the fiber material 4 and the linear elastic body 5a is enhanced, and therefore the occurrence of the "rubber dropout" when the linear elastic body 5a is cut can be prevented.

A thickness of the base fabric 1 can be arbitrarily set, and a thin base fabric can be formed. When the base fabric of the small thickness is formed, the air permeability and water permeability of the disposable textile product configured using the base fabric 1 can be improved and good feeling in wearing can be ensured.

When the paper material is used as the fiber material 4, the printed layer 4a can be formed, as necessary in the paper material, as depicted in Figs. 4 and 14. As depicted in Fig. 14, the printed layer 4a is formed in the fiber material 4 on the side facing the second fibrous sheet 3. In the embodiment of the present invention, printing can be performed on the paper material, rather than on the nonwoven fabric. Therefore, a clear picture can be printed easily and efficiently. As a result, various design and image can be represented efficiently and effectively. In particular, by selecting, as appropriate, the design or pattern to be printed on the paper material, the nonwoven fabric to be used in the second fibrous sheet 3, and a method for forming the adhesive layer 7, it is possible to provide the base fabric 1 as a whole with excellent design and aesthetic property. For example, a picture, color, pattern, photograph (referred to hereinbelow as "picture") are an example of the design and pattern to be printed on the paper material.

The picture can be printed on the paper material serving as the fiber material 4, for example, by ink-jet printing, flexographic printing, and gravure printing. The surface of the printed layer 4a may be subjected to a varnishing and discoloration prevention process, or a binder may be applied thereto. An example of the binder includes a well-known material such as PVA (polyvinyl alcohol), CMC (carboxymethyl cellulose), EVA (ethylene - vinyl acetate copolymer), acryl, and lacquer. An ink subjected to the discoloration preventing process can be also used.

It is preferred that the printed layer 4a be formed by flexographic printing. The advantage of the flexographic printing method is that the contact surface area between the plate and paper material can be small and a low pressure is applied during printing. Therefore, the paper material can be easily peeled off from the plate. Such a method is particularly advantageous for forming the printed layer 4a when the thickness of the paper material is small. Further, a large number of various type of ink can be used in the flexographic printing, and in this respect, the process is less affected by the ink viscosity than the ink-jet printing process in which the ink is discharged through a nozzle or the like. Where a picture is represented on the printed layer 4a, the picture which is to be printed by a plate can be printed as a picture of an elongated design according to the state in which the laminated sheet 30 is stretched in the extension/contraction direction (x direction in Fig. 2) of the linear elastic body 5a. When the tensioned state of the linear elastic body 5a is released, the picture of the original target design (non-elongated design) is displayed. In this case, the elongation ratio of the picture, which is to be printed, in the x direction is determined by the extension/contraction ratio of the linear elastic body 5a. For example, the printing can be performed by increasing the length of the picture with the original target design (non-elongated device) by a factor of 1.1 to 3.5.

A three-dimensional picture can be displayed on the printed layer 4a by forming a plurality of depression or protrusion on the paper material by the embossing process, and a picture of design that differs depending on the angle of view can be represented. Further, as depicted in Fig. 14, the printed layer 4a can be formed such as to be positioned in the space 9, and the printed layer 4a can be formed to be at positions covered by the adhesive layer 7. By combining such printed layer 4a, it is possible to expand the variety of design that can be represented on the product. Where the proportion of the space 9 is large, the printed layer 4a represented on the product appears to be somewhat blurred, and when the proportion of the space 9 is small, the printed layer 4a represented on the product is clearly visible.

Since the printed layer 4a is visible from the outside through the second fibrous sheet 3, various design or message can be efficiently and effectively represented on the base fabric 1. For the printed layer 4a to be accurately recognizable from the outside and to provide the product with high aesthetic property, it is preferred to use the nonwoven fabric as the second fibrous sheet 3.

In the disposable textile product configured using the base fabric 1 of the present invention, when a sweat 300 adheres to the skin 200, as depicted in Fig. 13a, where the body moves as depicted in Fig. 13b, the shirring portion 6 acts to wipe the sweat 300, and the sweat 300 which has thus been wiped is absorbed by the fiber material 4 (paper material) in the composite layer 31 (see Fig. 4) . Further, the sweat 300 that has not been wiped transpires into the space formed by the shirring portion 6 and is likewise absorbed by the fiber material 4 (paper material), as depicted in Fig. 13c. Accordingly, the base fabric 1 is not brought into intimate contact with the skin 200 by the sweat 300, and the sweat 300 absorbed by the fiber material 4 (paper material) is efficiently transpired to the outside (Fig. 13c). For this reason, the sweat 300 dries rapidly, there is no feeling of stickiness created by the sweat 300, and the product can be worn comfortably.

A variety of additive can be added to the base fabric 1 according to the property of the product when the base fabric 1 of the present invention is used in the disposable textile product. An example of the additive added to the base fabric 1 includes a deodorant, insect repellant, fragrance, waterproofing agent, antifouling agent, antibacterial agent, and softener, and those agents can be used individually, or in combination of two or more thereof. When added to the base fabric 1, the additive may be added to any one of the first fibrous sheet 2, the second fibrous sheet 3, and the fiber material 4, or may be added to two or more thereof. The addition may be made by applying the additive to the surface of the first fibrous sheet 2, the second fibrous sheet 3, and the fiber material 4. When the additive is added to the first fibrous sheet 2 and the second fibrous sheet 3, the additive may be kneaded in advance with the fiber which will constitute those sheets. In the case of addition to the paper material serving as the fiber material 4, the additive may be added in advance to water in the papermaking process for manufacturing the paper, thereby including the additive into the paper. Further, when the printed layer 4a is formed on the base fabric 1, the additive may be mixed in advance with the printing ink, and the printing ink including the additive may be printed.

A specific example of the deodorant includes catechin, epigallocatechin, gallocatechin, epicatechin gallate, epigallocatechin gallate, gallotannin, and ellagitannin which are extracts from plants, such as catechin and tannin; iron - ascorbic acid chelate compound; zirconium hydroxide; hydroxides of lanthanoid; salts of metal such as Zn, Cu, and Fe (for example, $ZnSO_4$). An example of the deodorant having an adsorptive action includes activated carbon; zeolite; silica; ceramic; Oya tuff stone; charcoal polymer; carbon nanotube; carbon nanohorn; organic acid such as citric acid and succinic acid; inorganic acid such as sulfuric acid, boric acid, and phosphoric acid; ion exchanger; anion, ammonia, amine, alkene, alkyne, a nucleophilic agent such as an aromatic compound; cation, boron fluoride, aluminum chloride, iron bromide, zinc chloride, and an electrophilic agent such as acetone. Those deodorants may be used individually or in combination of two or more thereof.

A specific example of insect repellant includes N, N-diethyl-m-toluamide (DEET), dipropyl pyridine-2,5-dicarboxylate, pyrethrin, dimethyl phthalate, 2,3:4,5-bis(2-butylene)tetrahydrofurfural, citronella, geraniol, lemon grass oil (essential oil), eugenol, p-menthane-3,8-diol, ethyl butyl acetyl aminopropionate, 1-piperidine carboxylic acid, 2-(2-hydroxy ethyl)-ester, and 1-methyl propyl-ester. In addition, natural plant essential oil, citronella oil, lemon grass oil, cinnamon oil, eucalyptus oil, clove oil, cinnamon oil, lemon eucalyptus oil, Hiba oil, lavender oil, orange oil, grapefruit oil, cedarwood oil, geranium oil, thyme white oil, and peppermint oil can be used as insect repellants. Furthermore, an essential oil including a component such as citronellal, citronellol, citral, linalool, dihydrolinalool, tetrahydrolinalool, dehydrolinalool, terpineol, menthol, menthane, p-menthane-3,8-diol, camphene, methyl salicylate, pinene, limonene, geraniol, borneol, and geranyl formate can be used as insect repellants. Those insect repellants may be used individually or in combination of two or more thereof.

A specific example of perfume includes (plant) an essential oil of fruit such as orange, lemon, lime, and peach; flower such as rose and lavender; and mint and sandalwood. An aromatic component is usually oil-based or soluble in water or alcohol. An example of oil-based perfume includes phenyl ethyl alcohol, linalool, jasmone, hexylcinnamic aldehyde, $\alpha$-linemon, $\alpha$-pinene, bromostyrene, citronellal, chloral, terpineol, menthol, and cinnamic acid. Those perfumes may be used individually or in combination of two or more thereof.

A carbendazim derivative with antimicrobial property, etc. can be used as the antibacterial agent.

An example of a suitable antifouling agent and softener includes a cationic surfactant such as alkylated quaternary ammonium salts which are widely used as a hair rinse and fabric softener. An example of such cationic surfactant includes dicocoyl dimethyl ammonium chloride and alkyl trimethyl ammonium chloride. Glycerin, propylene glycol, butylene glycol, dipropylene glycol, and liquid paraffin can be also used as the antifouling agent and softener. Those antifouling agents and softeners may be used individually or in combination of two or more thereof.

The base fabric 1 of the present invention is used as a material for the disposable textile product, and it is particularly beneficial when used as a constituent material of disposable undergarment such as disposable pants and disposable diaper. Another example of the disposable textile product that can be configured using the base fabric 1 of the present invention includes an undergarment or a T-shirt 100 such as depicted in Fig. 7, a tube top 101 such as depicted in Fig. 8, a belly band 102 such as depicted in Fig. 9, a rainwear 103 such as a raincoat depicted in Fig. 10, a supporter 104 that is used on various part, such as hand, arm, wrist, knee, and ankle, of the body as depicted in Figs. 11a to 11e, and a bandage 105 such as depicted in Fig. 12. Further, fitness wear, sportswear, sweat-absorbing inner wear, swimming wear, and room wear can be also used as the disposable textile product (such examples are not specifically illustrated by the figures). When the base fabric 1 of the present invention is used for a hat as the disposable textile product, it can be worn with the fiber material 4 impregnated with water. In this case, the evaporation of water produces a cooling effect on the head of the user.

The base fabric 1 of the present invention can be also used as a constituent material for a diaper bag for storing used diaper, various sanitary item for women, various disposable garment to be used in camping and outdoor activity, cooling or warming scarf, towel, and disposable clothing, bedding, long pants, bra, shorts, bathrobe, leg warmer, headband, hair band, massage clothes, and nursing clothing to be provided to patient and customer in a hospital, hotel, and the like.

Fig. 17 illustrates an embodiment in which a disposable undergarment 10 as the disposable textile product is configured using the base fabric 1 of the present invention. The undergarment 10 depicted in Fig. 17 is a diaper of the so-called pants-type which is used by wearing on the torso of a user P. This undergarment has a torso attachment part 12 which is configured of the base fabric 1 of the present invention, covers the entire torso of the user P, and includes a portion applying a light pressure to the lower abdomen, and a waist attachment portion 14 which is provided on the upper side of the torso attachment part 12, includes an elastic member such as rubber, and is attached around the waist of the user P. Inside the torso attachment part 12 (the side in contact with the body), an elastic sheet 16 is bridged between the front and rear portion of the torso attachment part 12, and inside the elastic sheet 16 (side in contact with the skin of the user P), an absorber 18 is attached for absorbing bodily wastes.

As indicated hereinabove, the undergarment 10 depicted in Fig. 17 functions as a diaper and can be used as a diaper for an infant and as a diaper for an adult. The diaper using the base fabric 1 of the present invention feels softer than the conventional diaper, excels in moisture transpiration ability, heat dissipation ability, and the moisture permeability, and advantageously creates a cold sensation. The undergarment 10 using the base fabric 1 of the present invention feels similarly to the usual undergarment (pants) when worn, and the specific feature thereof, which cannot be found in the conventional disposable diaper, is that it creates only weak sensation of resistance when used. Therefore, the undergarment can be used with confidence, without the sensation of resistance, as a disposable undergarment by the patient with a risk of incontinence, a woman giving birth at an advanced age, and a menstruating woman. When the paper material is used as the fiber material 4, which is the constituent material of the base fabric 1 of the present invention, generation of static electricity during use can be prevented because the paper material is hardly charged by friction.

The operation effect of the base fabric of the present invention configured in the above-described manner and the disposable textile product configured by using the base fabric of the present invention will be explained hereinbelow. Since the shirring portion 6 is formed on the laminated sheet 30 in the base fabric 1 of the present invention, the operation effect is such that the base fabric is soft in contact with the skin and the base fabric as a whole demonstrates the flexibility.

Further, the laminated sheet 30 includes the first fibrous sheet 2 having the air permeability, the second fibrous sheet 3 likewise having the air permeability, and the fiber material 4 having the liquid diffusibility as laminated constituent material. In such a configuration, the fiber layer having the air permeability and the fiber layer having the liquid diffusibility are present adjacently to each other. The composite layer 31 in which the fiber layer having the air permeability and the fiber layer having the liquid diffusibility are laminated adjacently to each other is formed in the laminated sheet 30. When the disposable textile product constituted by the base fabric 1 of the present invention is worn, for example, such that the first fibrous sheet 2 is in contact with the skin of the body, the sweat generated by the body is absorbed in the layer of the fiber material 4 through the first fibrous sheet 2, and the sweat is diffused inside the layer of the fiber material 4. The sweat which has diffused and widely dispersed inside the layer is transpired to the outside through the second fibrous sheet 3. Therefore, the transpiration of the sweat is performed efficiently and no sweat is locally retained in a region which is in contact with the base fabric.

Thus, since the air-permeable functional layer and the liquid-diffusible functional layer are present adjacently to each other in the composite layer 31 in the laminated sheet 30, the air-permeable functional layer and the liquid-diffusible functional layer interact, and the sweat transpiration efficiency is greatly increased by this interaction. Thus, since moisture passage in the air-permeable functional layer and moisture passage in the liquid-diffusible functional layer are arranged continuously and adjacently to each other, a very high efficiency of sweat absorption and drying is obtained. Further, since the shirring portion 6 formed on the laminated sheet 30 is configured to have a structure in which the uneven surface is formed by the composite layer 31, the air-permeable functional layer and the liquid-diffusible functional layer in the composite layer 31 are of an uneven shape, thereby ensuring a large surface area. As a result, the efficiency of sweat transpiration from the composite layer 31 is greatly increased.

The base fabric 1 of the present invention excels in heat dissipation ability and can efficiently dissipate the heat emanating from the body. The heat emanating from the body is transferred through the first fibrous sheet 2 to the layer of the fiber material 4, and this heat is diffused in the layer of the fiber material 4. The fiber material 4 has the liquid diffusibility, and because of the liquid diffusibility, the fiber material at the same time exhibits also heat diffusion ability. The heat which has diffused and widely dispersed in the layer is dissipated to the outside through the second fibrous sheet 3. Therefore, the heat is efficiently dissipated and no heat is retained between the body and the base fabric 1. Such excellent heat dissipation ability can be demonstrated in the base fabric 1 of the present invention because, in the same manner as in the above-described sweat transpiration action, the laminated sheet 30 has the composite layer 31 in which the fiber layer having the air permeability and the fiber layer having the liquid diffusibility are laminated adjacently to each other and the air-permeable functional layer and the liquid-diffusible function layer interact with each other in the composite layer 31.

The base fabric 1 of the present invention is also excellent in the moisture permeability. In a composite sheet formed by laminating a plurality of constituent material, the moisture permeability generally tends to decrease with the increase in the number of constituent layer. In the present invention, the material layer, namely, the fiber material 4, is added and therefore the number of constituent layer is increased by comparison with that in the usual laminate obtained by laminating two fibrous sheet. However, no decrease in the moisture permeability can be found in the base fabric 1 of the present invention as compared with the conventional product with good moisture permeability, and the same moisture permeability can be demonstrated as in the conventional product with good moisture permeability. The possibility of maintaining good moisture permeability despite the increase in the number of constituent layer can be said to be a specific effect of the present invention. The possibility of exhibiting such an effect can be attributed to the above-described configuration in which the laminated sheet 30 has the composite layer 31 in which the fiber layer having the air permeability and the fiber layer having the liquid diffusibility are laminated adjacently to each other, and the air-permeable functional layer and the liquid-diffusible function layer interact with each other in the composite layer 31.

When the fiber material 4 is the paper material in the base fabric 1 of the present invention, wherein the weakening process for the paper structure is performed by the embossing process of the paper material in the above-described manner, then the interaction of the air-permeable functional layer and the liquid-diffusible function layer can be greatly increased, and the moisture transpiration ability, heat dissipation ability, and the moisture permeability can be further improved.

Further, by performing printing on the base fabric 1 as an embodiment of the present invention, it is possible to represent a variety of design or message in an efficient and effective manner. The base fabric 1 of the embodiment of the present invention excels in the overall soft and plump feeling and also in the flexibility and can efficiently and effectively impart a variety of function, while utilizing the texture that is characteristic to the base fabric material itself. Another advantage of the base fabric 1 of the present invention is that it can be manufactured easily and at a low cost.

EXAMPLE

The present invention will be explained hereinbelow in greater detail with reference to a specific example of the stretchable composite sheet which is used in the present invention.

(Example 1)

A spunbonded nonwoven fabric was used as the air-permeable sheet, paper with a pulp content of 100% (sheet of paper for toilet paper: basis weight of 18 g/$m^2$) without printing was used as the liquid-diffusible fiber sheet, and urethane rubber having a diameter of 620 decitex was used as the linear elastic body. A sheet material with a total basis weight of 38 g/$m^2$ which was obtained by bonding the paper and urethane rubber with the hot-melt adhesive (total basis weight of the hot melt adhesive: 1.2 g/$m^2$) between two of the nonwoven fabric was taken as Example 1.

(Comparative Example 1)

A sheet material with the total basis weight of 20 g/$m^2$ which was obtained by using the nonwoven fabric and urethane rubber same as in Example 1, inserting the urethane rubber between each of the nonwoven fabric and bonding same with the hot-melt adhesive (total basis weight of the hot melt adhesive: 1.2 g/$m^2$) was taken as Comparative Example 1.

(Comparative Example 2)

A conventional sheet material with the total basis weight of 24 g/m$^2$ which was obtained by using a spunbonded nonwoven fabric on a front surface and a rear surface and inserting the urethane rubber having the diameter of 620 decitex between each of the nonwoven fabric was taken as Comparative Example 2.

(Comparative Example 3)

The conventional sheet material with the total basis weight of 22 g/m$^2$ which was obtained by using a thermally bonded nonwoven fabric on the front surface and rear surface and inserting the urethane rubber having the diameter of 620 decitex between each of the nonwoven fabric was taken as Comparative Example 3.

(Comparative Example 4)

Another conventional sheet material with the total basis weight of 22 g/m$^2$ which was obtained by using the thermally bonded nonwoven fabric on the front surface and rear surface and inserting the urethane rubber having the diameter of 620 decitex between each of the nonwoven fabric was taken as Comparative Example 4.

(Comparative Example 5)

The conventional sheet material with the total basis weight of 20 g/m$^2$ which was obtained by using the thermally bonded nonwoven fabric on the front surface and rear surface and inserting the urethane rubber having the diameter of 620 decitex between each of the nonwoven fabric was taken as Comparative Example 5.

Initially, moisture absorption and quick drying ability (transpiration ability) and the moisture permeability of the sheet material of Example 1 and Comparative Example 1 to 5 was evaluated.

The moisture absorption and quick drying ability was evaluated by combined evaluation of moisture-absorbing ability and quick drying ability by performing a transpiration (II) test (Boken standard BQEA028).

A testpiece with a diameter of about 9 cm was fabricated with respect to the sheet material of Example 1 and Comparative Example 1 to 5, and a mass (W) of each testpiece and a petri dish was measured. Then, 0.1 mL of water was dropped on the dish, the testpiece was placed thereon, and a mass (W0) was measured. The dish with the specimen was allowed to stay in a standard state (20°C, humidity 65% RH), and a mass (Wt) was measured after each predetermined interval of time (5 min, 10 min, and then after every 10 min up to 60 min). A transpiration rate (%) for each predetermined period of time was then calculated from the measured mass W, W0, Wt by using a following Equation (1).

$$\text{Transpiration rate (\%)} = \{(W0 - Wt)/(W0 - W)\} \times 100 \quad \dots(1)$$

Results are shown in Table 1.

Table 1

| | Transpiration rate (%) | | | | | | |
|---|---|---|---|---|---|---|---|
| | 5 min | 10 min | 20 min | 30 min | 40 min | 50 min | 60 min |
| Example 1 | 9.8 | 20.9 | 43 | 62.9 | 78.7 | 88.6 | 94.7 |
| Comparative Example 1 | 3.8 | 6.8 | 12.9 | 17.7 | 23.3 | 28.9 | 35.2 |
| Comparative Example 2 | 4.3 | 8.1 | 15.3 | 20.4 | 27.5 | 33 | 39.5 |
| Comparative Example 3 | 1.7 | 3 | 6.3 | 7.9 | 10.2 | 12.8 | 18.3 |
| Comparative Example 4 | 0.7 | 3.5 | 5.7 | 9.5 | 11.4 | 15.2 | 9 |
| Comparative Example 5 | 1.7 | 3.4 | 5.2 | 9.8 | 12.6 | 15.4 | 15.3 |

The result on the transpiration rate which are shown in Table 1 clearly indicates that in Comparative Example 3 to 5, the transpiration rate is 20% or less even after 60 min, and in Comparative Example 1 and 2, the transpiration rate is less than 20% after 20 min and 40% or less even after 60 min, whereas in Example 1, the transpiration rate exceeds 20% after 10 min, the transpiration rate exceeds 40% after 20 min, and then the transpiration rate exceeds 60% after 30 min, the transpiration rate exceeds 75% after 40 min, the transpiration rate exceeds 85% after 50 min, and the transpiration rate exceeds 90% after 60 min.

In the Boken standard BQEA028, as a measure of the evaluation, the preferred transpiration rate after 20 min from a start of the test is to be of 50% or higher for a woven and 40% or higher for a knitted product in the case of a sports application, and 40% or higher for a woven and 30% or higher for a knitted product in the case of a general application.

Therefore, since the sheet material of Example 1 makes it possible to obtain a transpiration rate of 40% or higher, it can be said that such material can be comfortably worn both in sports application and general application.

It follows from the above, that the disposable garment of the present invention that uses the sheet material of Example 1 has a very high moisture absorption and quick drying ability (transpiration ability).

The moisture permeability was then tested by the A-1 method (calcium chloride method) of JIS-L1099 (2012), and the moisture permeability ($g/m^2 \cdot h$) was determined and evaluated.

The moisture permeability is defined as a value obtained by calculating a mass (g) of water vapor permeating through the textile

A testpiece was sampled from the sheet material of Example 1 and Comparative Example 1 to 5 according to JISL0105 (6.3) (Cloth-like sample and the testpiece thereof)

A device and material such as a moisture-permeable cup, a thermostat-hygrostat, a round plate, and a moisture absorbent corresponding to the calcium chloride method were prepared and the test was performed.

Initially, about 33 g of the moisture absorbent was placed in the moisture-permeable cup which was warmed up to about 40°C, the moisture-permeable cup was vibrated to obtain a uniform material, the surface was leveled with a spatula, and a distance between the moisture absorbent and a lower surface of the testpiece was adjusted to 3 mm by using the round plate.

Then, three of the testpiece with a diameter of about 70 mm were sampled according to JISL0105 (6.3) (cloth-like sample and the testpiece thereof) with respect to the sheet material of Example 1 and Comparative Example 1 to 5.

Each testpiece was placed to be concentric with the moisture-permeable cup, such that the surface of the testpiece faced the moisture absorbent, a packing and a ring were successively mounted and fixed with a wing nut, and a mounting-side surface was sealed with a vinyl pressure-sensitive adhesive tape to form a test sample. The test sample was placed at a location inside the thermostat-hygrostat with a temperature of 40°C ± 2°C and a humidity (90 ± 5) % RH, such that the air speed about 10 mm above the testpiece did not exceed 0.8 m/s. The test sample was taken out after 1 h and a mass (a1) was immediately measured with an accuracy up to 1 mg. After the measurement, the test sample was again placed at the same location in the thermostat-hygrostat, the test sample was taken out after 1 h, and a mass (a2) was immediately measured with an accuracy up to 1 mg. The moisture permeability PA1 ($g/m^2 \cdot h$) was then calculated from the measured mass a1 and a2 by using a following Equation (2).

$$\text{Moisture permeability PA1 } (g/m^2 \cdot h) = (a2 - a1)/SA1 \quad \ldots (2)$$

In Equation (2), a2 - a1 is the change amount (g/h) of a mass of the test sample per 1 h, and SA1 is the moisture permeation area ($m^2$).

The test result was rounded to an integer by a three mean value method stipulated by JISZ8401 (Rounding method). The obtained result is shown in Table 2.

Table 2

| | Moisture permeability ($g/m^2 \cdot h$) |
|---|---|
| Example 1 | 564 |
| Comparative Example 1 | 563 |
| Comparative Example 2 | 435 |
| Comparative Example 3 | 496 |
| Comparative Example 4 | 526 |
| Comparative Example 5 | 433 |

The result relating to the moisture permeability which is shown in Table 2 clearly indicates that the moisture permeability is 500 g/$m^2$·h or less in Comparative Example 2, 3, and 5, 563 g/$m^2$·h in Comparative Example 1, and 526 g/$m^2$·h in Comparative Example 4. Meanwhile, in Example 1, the moisture permeability is 564 g/$m^2$·h which is higher than in the comparative example.

It follows from the above that the functional base fabric material of the present invention is highly stable and has good moisture permeability.

The moisture permeability evaluation result and transpiration evaluation result after 20 min and 60 min are shown in Table 3 with respect to Example 1 and Comparative Example 1 to 5.

The moisture permeability evaluation result and transpiration evaluation result shown in Table 3 clearly demonstrate that the functional base fabric material of the present invention which is represented by Example 1 is superior to the functional base fabric material of Comparative Example 1 to 5 in both the moisture permeability and the transpiration ability and makes it possible to obtain higher moisture permeability and transpiration rate.

The result presented hereinabove clearly demonstrates the effect of the functional base fabric material for a disposable product and of the disposable fabric product in accordance with the present invention.

Table 3

| | Moisture permeability (g/$m^2$·h) | Transpiration ability (%) after 20 min | Transpiration ability (%) after 60 min | Total basis weight (g/$m^2$) |
|---|---|---|---|---|
| Example 1 | 564 | 43 | 94.7 | 149.3 |
| Comparative Example 1 | 563 | 12.9 | 35.2 | 92.3 |
| Comparative Example 2 | 435 | 15.3 | 39.5 | 123.2 |
| Comparative Example 3 | 496 | 6.3 | 18.3 | 144.9 |
| Comparative Example 4 | 526 | 5.7 | 17.9 | 147.8 |
| Comparative Example 5 | 433 | 5.2 | 15.3 | 143 |

It goes without saying that the present invention is not limited to the above-described embodiment and is inclusive of all embodiment making it possible to attain the object of the present invention.

Cool contact feeling (cool feeling) and wet contact cold feeling (stickiness during perspiration) of the sheet material in which paper is interposed between each of the nonwoven fabric and a sheet material in which no paper is interposed between each of the nonwoven fabric were then tested.

(Example 2 to 5)

The sheet material was prepared by using the nonwoven fabric, paper, hot-melt adhesive, and urethane rubber similar to those of Example 1. The paper used in Example 2 and 3 was subjected to the embossing process the number of cycles shown in Table 4, by using the pair of the flat roll. The basis weight of the paper used, the total basis weight of the hot-melt adhesive, and the total basis weight of the sheet material are all shown in Table 4. The cool contact feeling and wet contact hot feeling were measured by the below-described method with respect to the sheet material. A value of the cool contact feeling, wet contact cold feeling, and the ratio of the cool contact feeling and wet contact cold feeling [(cool contact feeling)/(wet contact cold feeling)] is shown in Table 5.

Cool contact feeling: the cool contact feeling represents the degree of sensation of cooling and refreshing in wearing, and it was measured by the following method by using a precision rapid thermal physical property measurement device (KES-F7 Thermolab II, manufactured by Kato Tech Co., Ltd.).

The testpiece (6 cm × 6 cm) cut out from the sheet material was placed on a base plate at a temperature of 20°C. A hot plate equipped with a precision thermal sensor was heated to 40°C, the hot plate was placed on the testpiece at 20°C, and the cool contact feeling value (qmax) was calculated from the heat dissipation behavior measured with the thermal sensor of the hot plate.

Wet contact cold feeling: the wet contact cold feeling represents the degree of sticky feeling when sweating, and it was measured by the following method by using the precision rapid thermal physical property measurement device (KES-F7 Thermolab II, manufactured by Kato Tech Co., Ltd.).

The testpiece (6 cm × 6 cm) cut out from the sheet material was caused to absorb water at 80 g/$m^2$, and the resultant testpiece carrying water was placed on the base plate at a temperature of 20°C. The hot plate equipped with a precision thermal sensor was heated to 40°C, the hot plate was placed on the testpiece at 20°C, and the wet contact cold feeling value (wet-qmax) was calculated from the heat dissipation behavior measured with the thermal sensor of the hot plate.

(Comparative Example 6)

The cool contact feeling and wet contact cold feeling were tested in the same manner as in Example 2 to 5 by using the sheet material similar to that of Comparative Example 2. The result is shown in Table 5.

(Comparative Example 7)

The cool contact feeling and wet contact cold feeling were tested in the same manner as in Example 2 to 5 by using the sheet material similar to that of Comparative Example 4. The result is shown in Table 5.

(Comparative Example 8)

The cool contact feeling and wet contact cold feeling were tested in the same manner as in Example 2 to 5 by using the sheet material similar to that of Comparative Example 3. The result is shown in Table 5.

(Comparative Example 9)

The cool contact feeling and wet contact cold feeling were tested in the same manner as in Example 2 to 5 by using the sheet material similar to that of Comparative Example 5. The result is shown in Table 5.

Table 4

| | Total basis weight (g/$m^2$) | Basis weight of hot-melt adhesive (g/$m^2$) | Number of embossing cycles (cycle) | Basis weight of sheet material (g/$m^2$) |
|---|---|---|---|---|
| Example 2 | 18 | 1.2 | 1 | 150.8 |
| Example 3 | 18 | 1.2 | 2 | 148.1 |
| Example 4 | 18 | 1.2 | - | 146.2 |
| Example 5 | 13 | 1.2 | - | 137.4 |

Table 5

| | Cool contact feeling | Wet contact cold feeling | (Cool contact feeling)/(wet contact cold feeling) | Total basis weight (g/$m^2$) |
|---|---|---|---|---|
| Example 2 | 45 | 91 | 0.49 | 150.8 |
| Example 3 | 49 | 95 | 0.52 | 148.1 |
| Example 4 | 42 | 90 | 0.47 | 146.2 |
| Example 5 | 50 | 96 | 0.52 | 137.4 |
| Comparative Example 6 | 46 | 164 | 0.28 | 123.2 |
| Comparative Example 7 | 37 | 165 | 0.22 | 144.9 |

(continued)

| | Cool contact feeling | Wet contact cold feeling | (Cool contact feeling)/(wet contact cold feeling) | Total basis weight ($g/m^2$) |
|---|---|---|---|---|
| Comparative Example 8 | 38 | 225 | 0.17 | 147.8 |
| Comparative Example 9 | 27 | 119 | 0.23 | 143.0 |

[Reference Signs List]

2 First fibrous sheet
3 Second fibrous sheet
4 Fiber material
5 Elastic member

**Claims**

1. Abase fabric for a disposable textile product configured of a laminated sheet which has a first fibrous sheet and a second fibrous sheet having air permeability and a fiber material interposed between the first fibrous sheet and the second fibrous sheet and having liquid diffusibility, and in which the first fibrous sheet and the second fibrous sheet and the fiber material are laminated together with an elastic member, **characterized in that**
the laminated sheet forms a composite layer in which a fiber layer having the air permeability and a fiber layer having the liquid diffusibility are laminated;
the laminated sheet has a shirring portion in which an uneven surface is formed by the composite layer; and elasticity is imparted to the laminated sheet,
wherein the first fibrous sheet and the second fibrous sheet are configured of nonwoven fabric, wherein the basis weight of the nonwoven fabric used for the first fibrous sheet and the second fibrous sheet is 5 $g/m^2$ to 60 $g/m^2$,
wherein the fiber material is configured of a paper material,
wherein the basis weight of the paper material constituting the fiber material is 7 $g/m^2$ to 50 $g/m^2$
wherein the first fibrous sheet and the fiber material are intermittently joined together through the elastic member,
wherein the second fibrous sheet and the fiber material are intermittently joined together.

2. The base fabric for the disposable textile product according to claim 1, wherein the elasticity is imparted to the laminated sheet by the elastic member provided between the first fibrous sheet and the fiber material.

3. The base fabric for the disposable textile product according to any one of claims 1 to 2, wherein the first fibrous sheet and the fiber material are joined together by a hot-melt adhesive through the elastic member.

4. The base fabric for the disposable textile product according to any one of claims 1 to 3, wherein the second fibrous sheet and the fiber material are joined together by a hot-melt adhesive.

5. The base fabric for the disposable textile product according to any one of claims 1 to 4, wherein the elastic member is configured of a plurality of linear elastic body having stretching ability, and the plurality of linear elastic body is disposed at an interval in a width direction of the laminated sheet and joined between the first fibrous sheet and the fiber material.

6. The base fabric for the disposable textile product according to claim 5, wherein the linear elastic body is provided over an entire region or in a partial region inside the laminated sheet.

7. The base fabric for the disposable textile product according to claim 5 or 6, wherein, in the laminated sheet, a plurality of shirring portion extending in a direction perpendicular to a longitudinal direction of the linear elastic body in a non-tensioned state is formed, and a shirring row is formed in a pattern.

8. The base fabric for the disposable textile product according to any one of claims 1 to 7, wherein the fiber material has a flexible structure obtained by a mechanical softening process.

9. The base fabric for the disposable textile product according to any one of claims 1 to 8, wherein the fiber material is subjected to a weakening process.

10. The base fabric for the disposable textile product according to any one of claims 1 to 9, wherein the first fibrous sheet and the second fibrous sheet are configured of a material having the air permeability and moisture permeability.

11. The base fabric for the disposable textile product according to any one of claims 1 to 10, wherein the fiber material is configured of a material having the liquid diffusibility and liquid permeability.

12. A disposable textile product configured using the base fabric for a disposable product according to any one of claims 1 to 11.

13. The disposable textile product according to claim 12, wherein
the disposable textile product is configured as an undergarment, a diaper, a fitness wear, a swimming wear, a tube top, a room wear, a raincoat, or a belly band.

14. The disposable textile product according to claim any one of claims 1 to 13, wherein when a transpiration test of the laminated sheet is performed according to a BokenIlmethod by measuring a total weight (w) of the laminated sheet and a petri dish in the standard state; dropping 0.1mL of water on the petri dish and the laminated sheet; measuring a total mass (W0) of the petri dish, 0.1mL of water dropped on the petri dish, and the laminated sheet on the petri dish; and then measuring a mass (Wt) of the petri dish on which the water and the laminated sheet are placed after each predetermined interval of time from the dropping of the 0.1mL of water, the transpiration rate determined by the following equation is 40% or higher after 20 minutes:

$$\text{Transpiration rate (\%)} = \{(W0- Wt)/( W0- W)\}\times 100.$$

**Patentansprüche**

1. Grundtextilstoff für ein wegwerfbares Textilprodukt, das aus einem laminierten Blatt konfiguriert ist, das ein erstes faseriges Blatt und ein zweites faseriges Blatt, das Luftdurchlässigkeit aufweist, und ein Fasermaterial, das zwischen dem ersten faserigen Blatt und dem zweiten faserigen Blatt eingeschoben ist und Flüssigkeitsdiffusionsvermögen aufweist, aufweist, und wobei das erste faserige Blatt und das zweite faserige Blatt und das Fasermaterial mit einem elastischen Element zusammenlaminiert sind, **dadurch gekennzeichnet, dass**
das laminierte Blatt eine Verbundstoffschicht bildet, in der eine Faserschicht, die die Luftdurchlässigkeit aufweist, und eine Faserschicht, die das Flüssigkeitsdiffusionsvermögen aufweist, lamininiert sind;
das lamininierte Blatt einen Kräuselabschnitt aufweist, in dem eine ungleichmäßige Oberfläche durch die Verbundstoffschicht gebildet ist; und
Elastizität dem laminierten Blatt verliehen wird, wobei das erste faserige Blatt und das zweite faserige Blatt aus Vliesstoff konfiguriert sind, wobei das Flächengewicht des Vliesstoffs, der für das erste faserige Blatt und das zweite faserige Blatt verwendet wird, 5 g/m$^2$ bis 60 g/m$^2$ beträgt,
wobei das Fasermaterial aus einem Papiermaterial konfiguriert ist, wobei das Flächengewicht des Papiermaterials, das das Fasermaterial bildet, 7 g/m$^2$ bis 50 beträgt,
wobei das erste faserige Blatt und das Fasermaterial diskontinuierlich durch das elastische Element miteinander verbunden sind, wobei das zweite faserige Blatt und das Fasermaterial diskontinuierlich miteinander verbunden sind.

2. Grundtextilstoff für das wegwerfbare Textilprodukt nach Anspruch 1, wobei die Elastizität dem laminierten Blatt durch das elastische Element verliehen wird, das zwischen dem ersten faserigen Blatt und dem Fasermaterial bereitgestellt ist.

3. Grundtextilstoff für das wegwerfbare Textilprodukt nach irgendeinem der Ansprüche 1 bis 2, wobei das erste faserige Blatt und das Fasermaterial durch einen Heißschmelzklebstoff durch das elastische Element miteinander verbunden sind.

4. Grundtextilstoff für das wegwerfbare Textilprodukt nach irgendeinem der Ansprüche 1 bis 3, wobei das zweite faserige Blatt und das Fasermaterial durch einen Heißschmelzklebstoff miteinander verbunden sind.

5. Grundtextilstoff für das wegwerfbare Textilprodukt nach irgendeinem der Ansprüche 1 bis 4, wobei das elastische Element aus mehreren linearen elastischen Körpern konfiguriert ist, die Dehnbarkeit aufweisen, und die mehreren linearen elastischen Körper in einem Abstand in einer Breitenrichtung des laminierten Blatts angeordnet und zwischen dem ersten faserigen Blatt und dem Fasermaterial verbunden sind.

6. Grundtextilstoff für das wegwerfbare Textilprodukt nach Anspruch 5, wobei der lineare elastische Körper über eine gesamte Region oder in einer Teilregion innerhalb des laminierten Blatts bereitgestellt ist.

7. Grundtextilstoff für das wegwerfbare Textilprodukt nach Anspruch 5 oder 6, wobei, in dem laminierten Blatt, mehrere Kräuselabschnitte, die sich in einer Richtung senkrecht zu einer Längsrichtung des linearen elastischen Körpers erstrecken, in einem nichtgespannten Zustand gebildet sind, und eine Kräuselreihe in einem Muster gebildet ist.

8. Grundtextilstoff für das wegwerfbare Textilprodukt nach irgendeinem der Ansprüche 1 bis 7, wobei das Fasermaterial eine flexible Struktur aufweist, die durch einen mechanischen Erweichungsvorgang erhalten wird.

9. Grundtextilstoff für das wegwerfbare Textilprodukt nach irgendeinem der Ansprüche 1 bis 8, wobei das Fasermaterial einem Abschwächungsvorgang unterworfen wird.

10. Grundtextilstoff für das wegwerfbare Textilprodukt nach irgendeinem der Ansprüche 1 bis 9, wobei das erste faserige Blatt und das zweite faserige Blatt aus einem Material konfiguriert sind, das Luftdurchlässigkeit und Feuchtigkeitsdurchlässigkeit aufweist.

11. Grundtextilstoff für das wegwerfbare Textilprodukt nach irgendeinem der Ansprüche 1 bis 10, wobei das Fasermaterial aus einem Material konfiguriert ist, das Flüssigkeitsdiffusionsvermögen und Flüssigkeitsdurchlässigkeit aufweist.

12. Wegwerfbares Textilprodukt, das unter Anwendung des Grundtextilstoffs für ein wegwerfbares Textilprodukt nach irgendeinem der Ansprüche 1 bis 11 konfiguriert ist.

13. Wegwerfbares Textilprodukt nach Anspruch 12, wobei
das wegwerfbare Textilprodukt als Unterwäsche, eine Windel, eine Fitnessbekleidung, eine Badebekleidung, einen Schlauch-Top, eine Heimbekleidung, einen Regenmantel oder eine Bauchbinde konfiguriert ist.

14. Wegwerfbares Textilprodukt nach irgendeinem der Ansprüche 1 bis 13, wobei, wenn ein Transpirationstest an dem laminierten Blatt dem Boken II-Verfahren entsprechend ausgeführt wird durch Messen eines Gesamtgewichts (w) des laminierten Blatts und einer Petrischale im Standardzustand; Tropfenlassen von 0,1 ml Wasser auf die Petrischale und das laminierte Blatt; Messen einer Gesamtmasse (W0) der Petrischale, der 0,1 ml des auf die Petrischale getropften Wassers und des laminierten Blatts auf der Petrischale; und dann Messen einer Masse (Wt) der Petrischale, auf der das Wasser und das laminierte Blatt positioniert sind, nach einer vorbestimmten Zeitspanne vom Tropfenlassen der 0,1 ml Wasser, die Transpirationsrate, die durch die folgende Gleichung bestimmt wird, nach 20 Minuten 40 % oder mehr beträgt:

$$\text{Transpirationsrate (\%)} = \{(W0 - Wt / (WO - W)\} \times 100.$$

## Revendications

1. Tissu de base pour un produit textile jetable constitué d'un stratifié en planche qui comporte une première feuille fibreuse et une seconde feuille fibreuse ayant une perméabilité à l'air et un matériau de fibre interposé entre la première feuille fibreuse et la seconde feuille fibreuse et ayant une diffusibilité aux liquides, et dans lequel la première feuille fibreuse et la seconde feuille fibreuse et le matériau de fibre sont stratifiés ensemble avec un élément élastique, **caractérisé en ce que**
le stratifié en planche forme une couche composite dans laquelle une couche de fibre ayant la perméabilité à l'air et une couche de fibre ayant une diffusibilité aux liquides sont stratifiées ;
le stratifié en planche comporte une partie de fronçage dans laquelle une surface inégale est formée par la couche composite ; et
l'élasticité est transmise au stratifié en planche, dans lequel la première feuille fibreuse et la seconde feuille fibreuse sont constituées d'un tissu non tissé, dans lequel la masse surfacique du tissu non tissé utilisé pour la première feuille fibreuse et pour la deuxième feuille fibreuse est comprise entre 5 et 60 g/m$^2$,
dans lequel le matériau de fibre est constitué d'un matériau en papier, dans lequel la masse surfacique du matériau en papier constituant le matériau de fibre est comprise entre 7 et 50 g/m$^2$,
dans lequel la première feuille fibreuse et le matériau de fibre sont joints ensemble de manière intermittente grâce à l'élément élastique, dans lequel la seconde feuille fibreuse et le matériau de fibre sont joints ensemble de manière intermittente.

**2.** Tissu de base pour un produit textile jetable selon la revendication 1, dans lequel l'élasticité est transférée au stratifié en planche par l'élément élastique monté entre la première feuille fibreuse et le matériau de fibre.

**3.** Tissu de base pour un produit textile jetable selon la revendication 1 ou la revendication 2, dans lequel la première feuille fibreuse et le matériau de fibre sont joints ensemble par un adhésif thermofusible grâce à l'élément élastique.

**4.** Tissu de base pour un produit textile jetable selon l'une quelconque des revendications 1 à 3, dans lequel la seconde feuille fibreuse et le matériau de fibre sont joints ensemble par un adhésif thermofusible.

**5.** Tissu de base pour un produit textile jetable selon l'une quelconque des revendications 1 à 4, dans lequel l'élément élastique est constitué d'une pluralité de corps élastiques linéaires ayant une capacité d'élasticité, et la pluralité de corps élastiques linéaires sont disposés à un certain intervalle dans une direction de largeur du stratifié en planche et sont joints entre la première feuille fibreuse et le matériau de fibre.

**6.** Tissu de base pour un produit textile jetable selon la revendication 5, dans lequel le corps élastique linéaire est monté sur une région complète ou dans une région partielle à l'intérieur du stratifié en planche.

**7.** Tissu de base pour un produit textile jetable selon la revendication 5 ou la revendication 6, dans lequel, dans le stratifié en planche, une pluralité de parties de fronçage s'étendant dans une direction perpendiculaire à une direction longitudinale du corps élastique linéaire dans un état de non-tension est formée, et une rangée de fronçage est formée selon un motif.

**8.** Tissu de base pour un produit textile jetable selon l'une quelconque des revendications 1 à 7, dans lequel le matériau de fibre comporte une structure flexible obtenue par un processus de ramollissement mécanique.

**9.** Tissu de base pour un produit textile jetable selon l'une quelconque des revendications 1 à 8, dans lequel le matériau de fibre est assujetti à un processus d'affaiblissement.

**10.** Tissu de base pour un produit textile jetable selon l'une quelconque des revendications 1 à 9, dans lequel la première feuille fibreuse et la seconde feuille fibreuse sont constituées d'un matériau ayant une perméabilité à l'air et une perméabilité à l'humidité.

**11.** Tissu de base pour un produit textile jetable selon l'une quelconque des revendications 1 à 10, dans lequel le matériau de fibre est constitué d'un matériau ayant une diffusibilité aux liquides et une perméabilité aux liquides.

**12.** Produit textile jetable conçu en utilisant le tissu de base pour un produit jetable conformément à l'une quelconque des revendications 1 à 11.

**13.** Produit textile jetable selon la revendication 12, dans lequel
le produit textile jetable est conçu comme un sous-vêtement, une couche, un vêtement de remise en forme, un maillot de bain, un bustier tubulaire, un vêtement de chambre, un imperméable, ou une sous-ventrière.

**14.** Produit textile jetable selon l'une quelconque des revendications 1 à 13, dans lequel un test de transpiration du stratifié en planche est effectué conformément à un procédé Boken II en mesurant un poids total (W) du stratifié en planche et une boîte de Petri dans un état standard ; en versant au goutte-à-goutte 0,1 ml d'eau sur la boîte de Petri et sur le stratifié en planche ; en mesurant une masse totale (W0) de la boîte de Petri, 0,1 ml d'eau versée goutte-à-goutte sur la boîte de Petri, et le stratifié en planche sur la boîte de Pétri ; et ensuite en mesurant une masse (Wt) de la boîte de Petri sur laquelle l'eau et le stratifié en planche sont placés après chaque intervalle temporel prédéterminé à partir du versement au goutte-à-goutte de 0,1 ml d'eau, le taux de transpiration déterminé par l'équation suivante est supérieur ou égal à 40 % après 20 minutes :

$$\text{Taux de transpiration (\%)} = \{(W0 - Wt) / (W0 - W)\} \times 100.$$

Fig.1

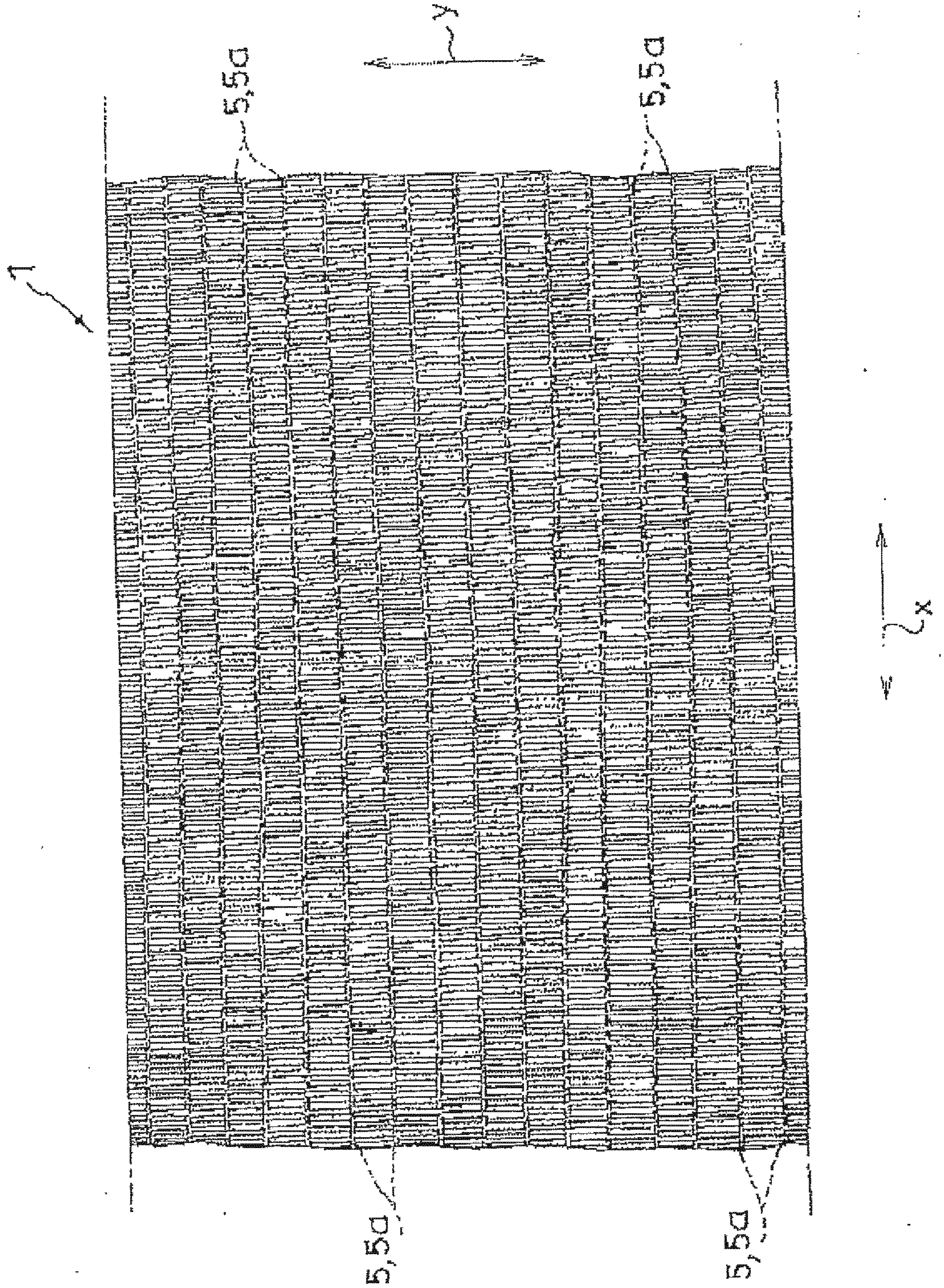

Fig.2

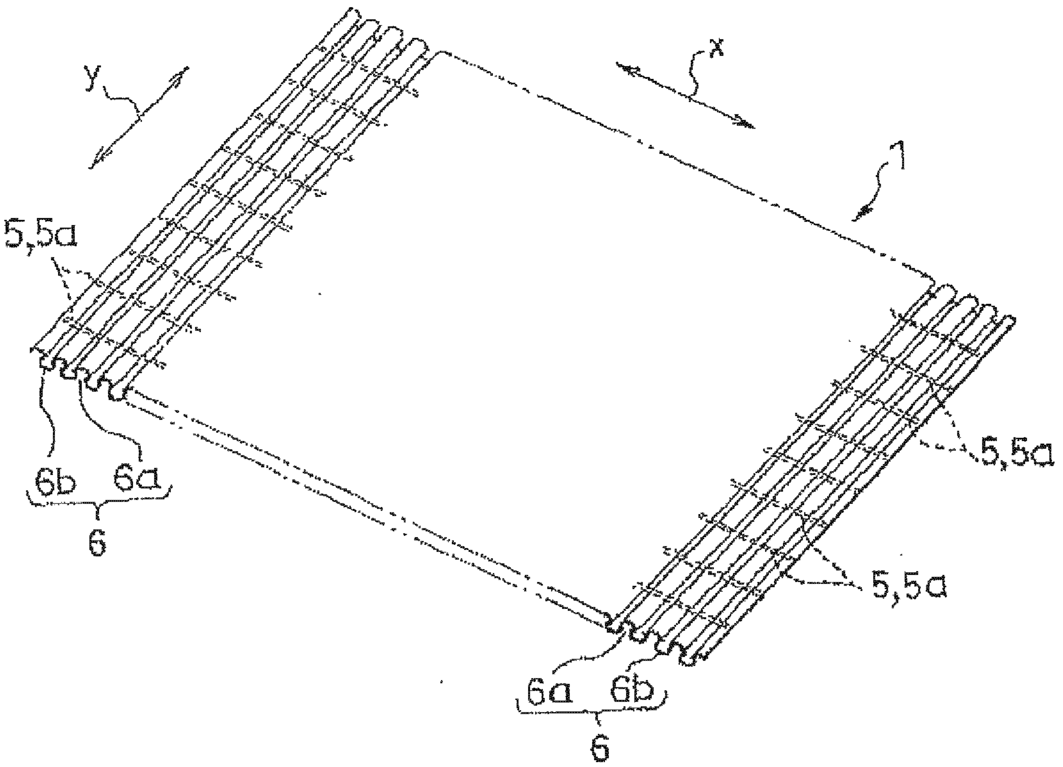

Fig.3

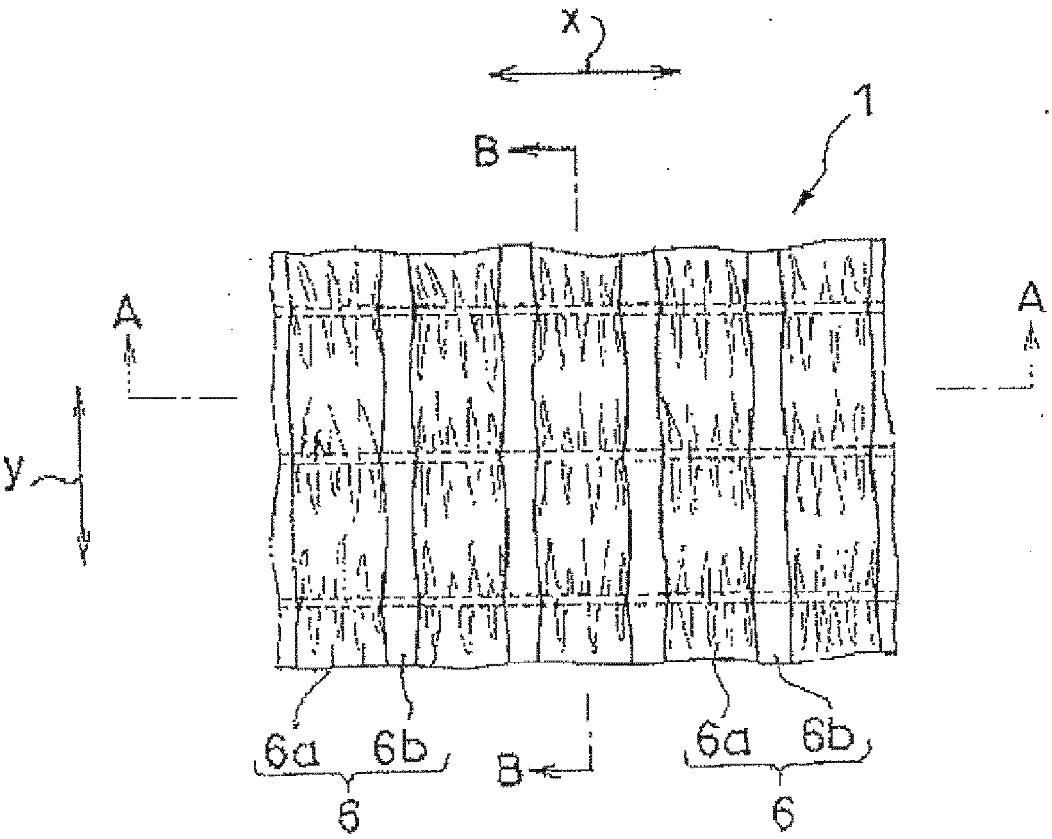

Fig.4

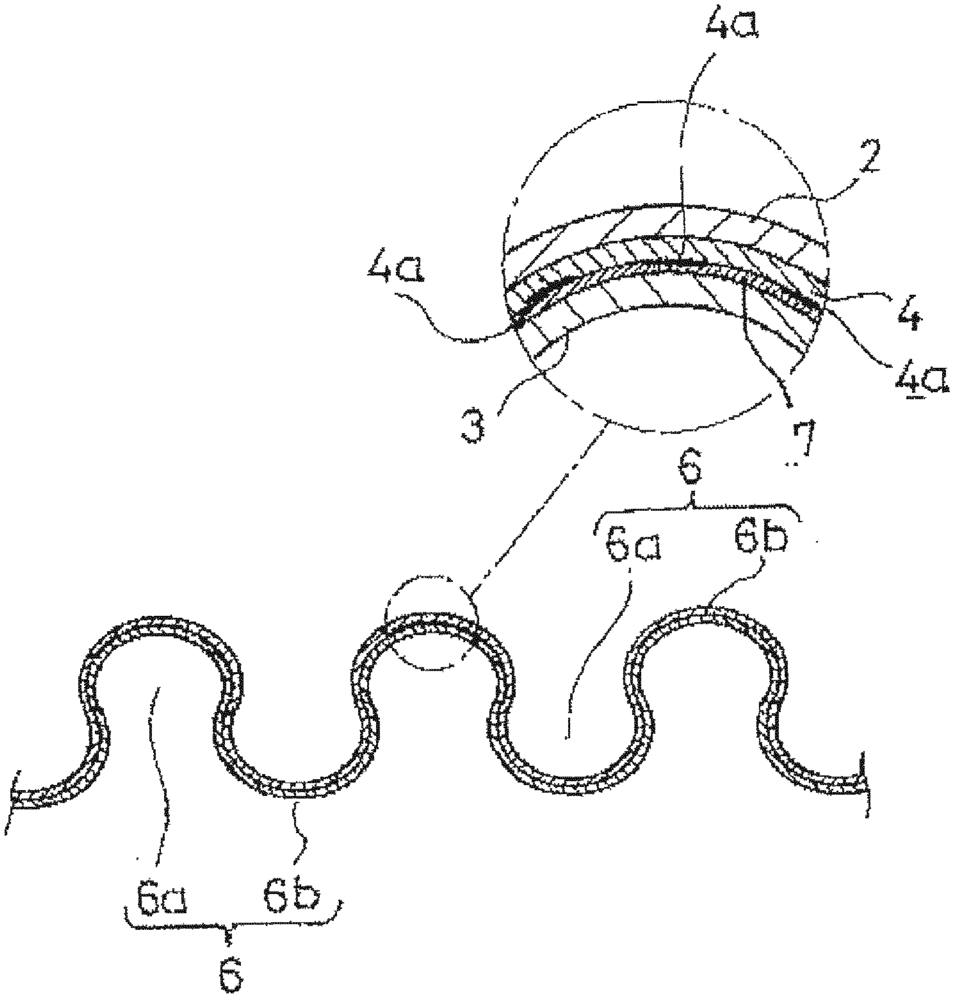

Fig.5

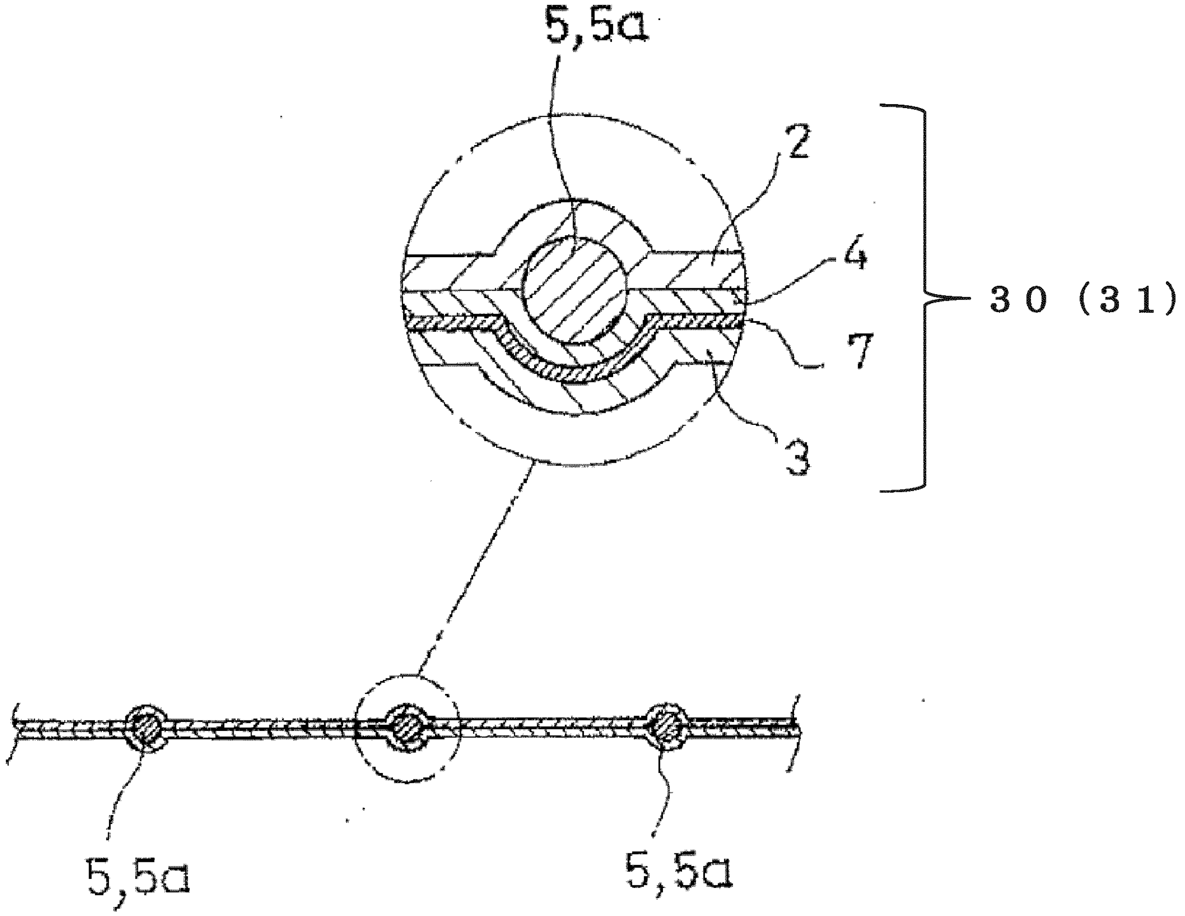

Fig.6

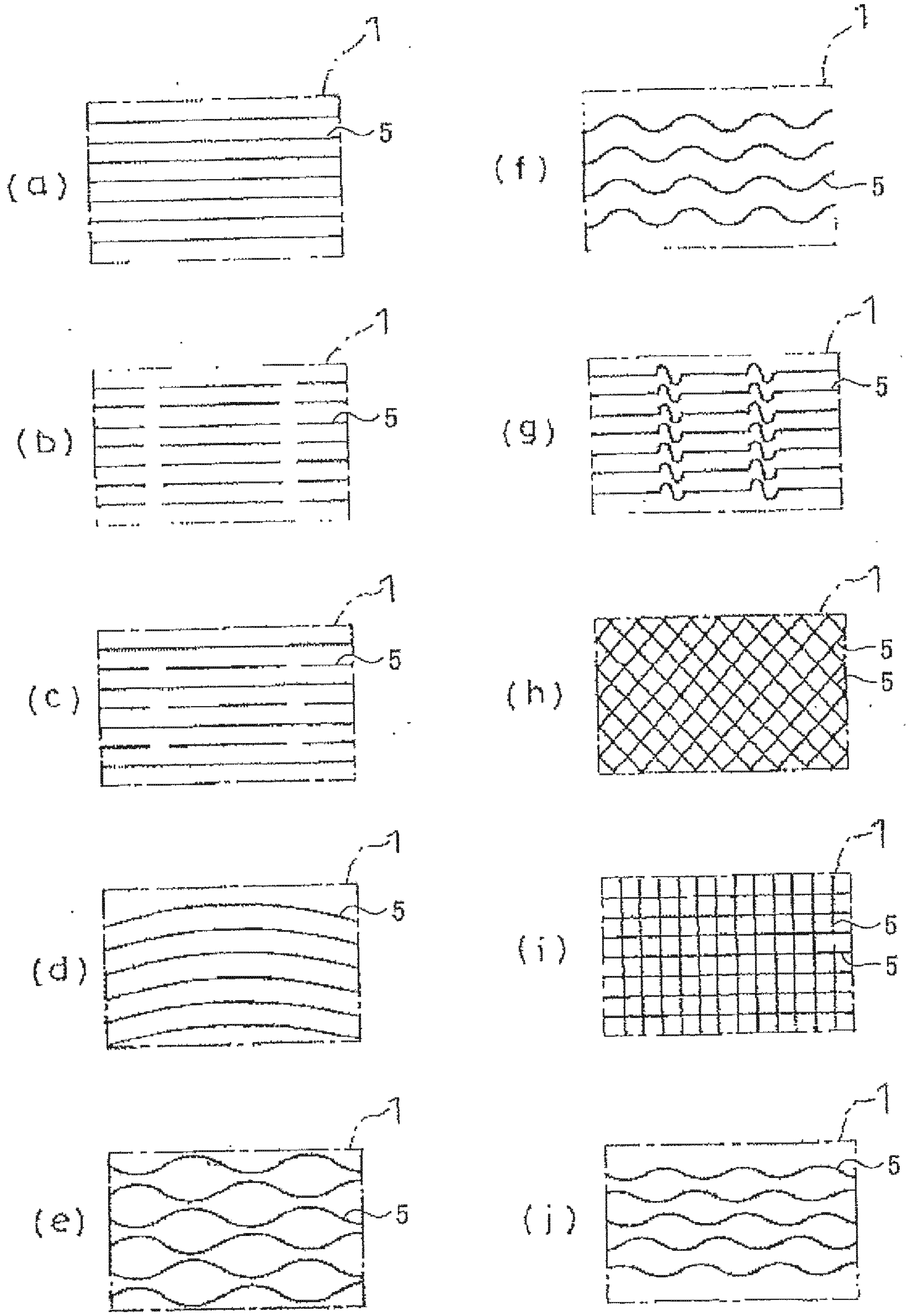

Fig.7

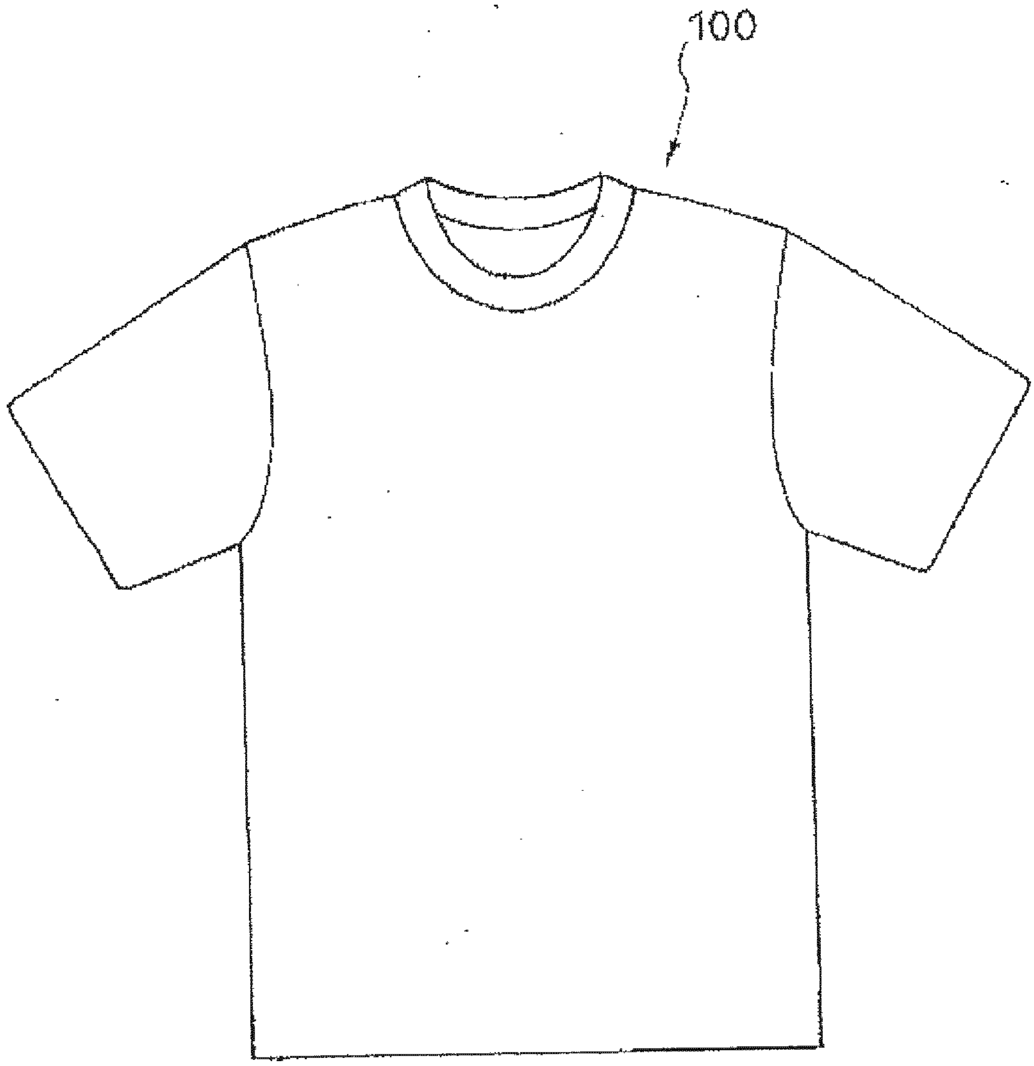

Fig.8

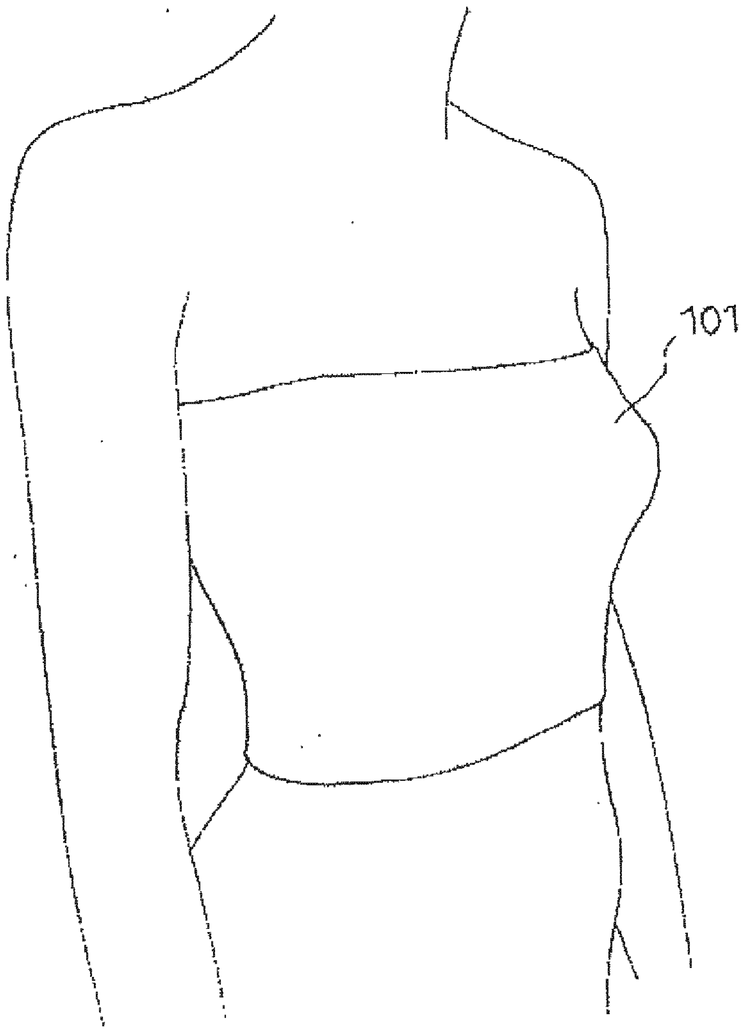

Fig.9

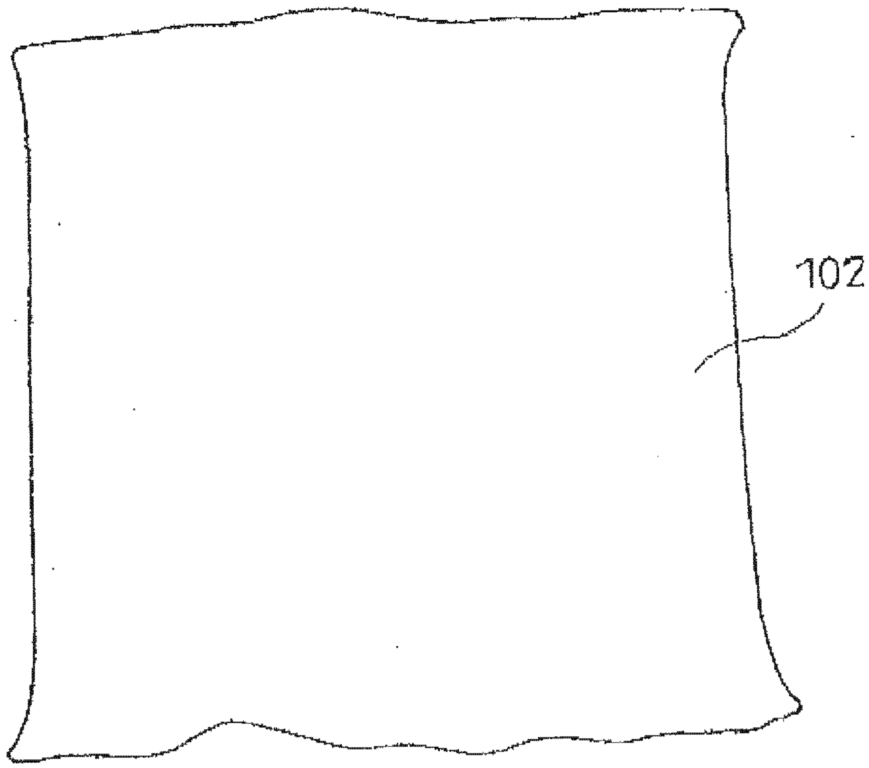

Fig.10

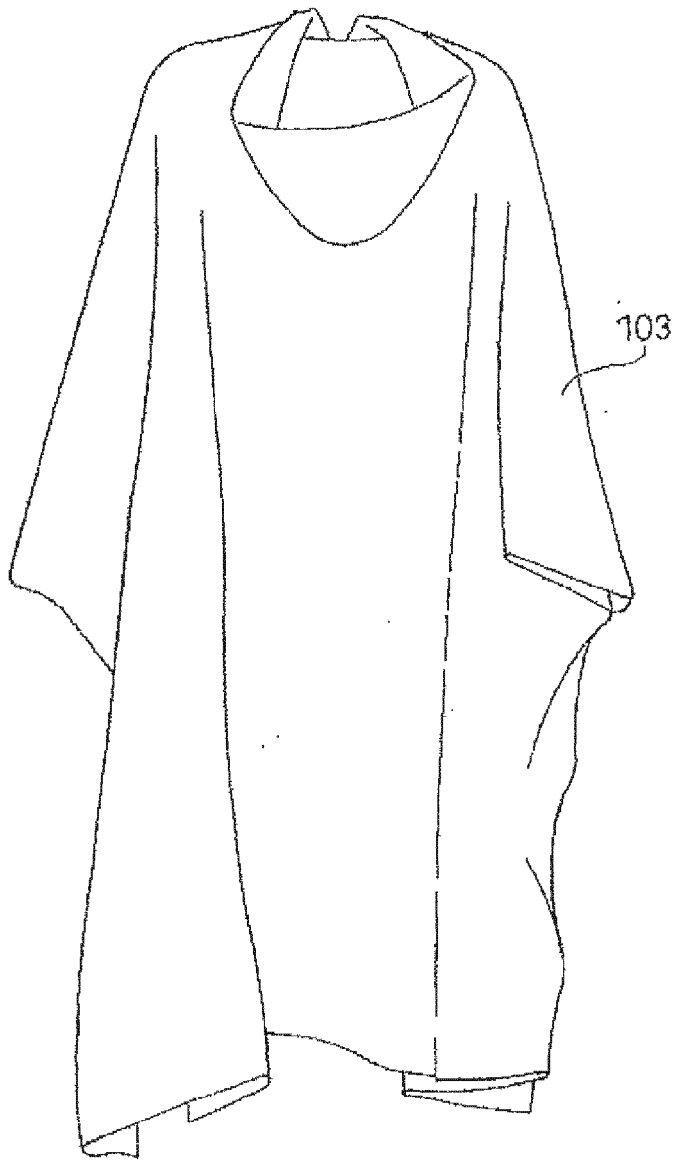

Fig.11

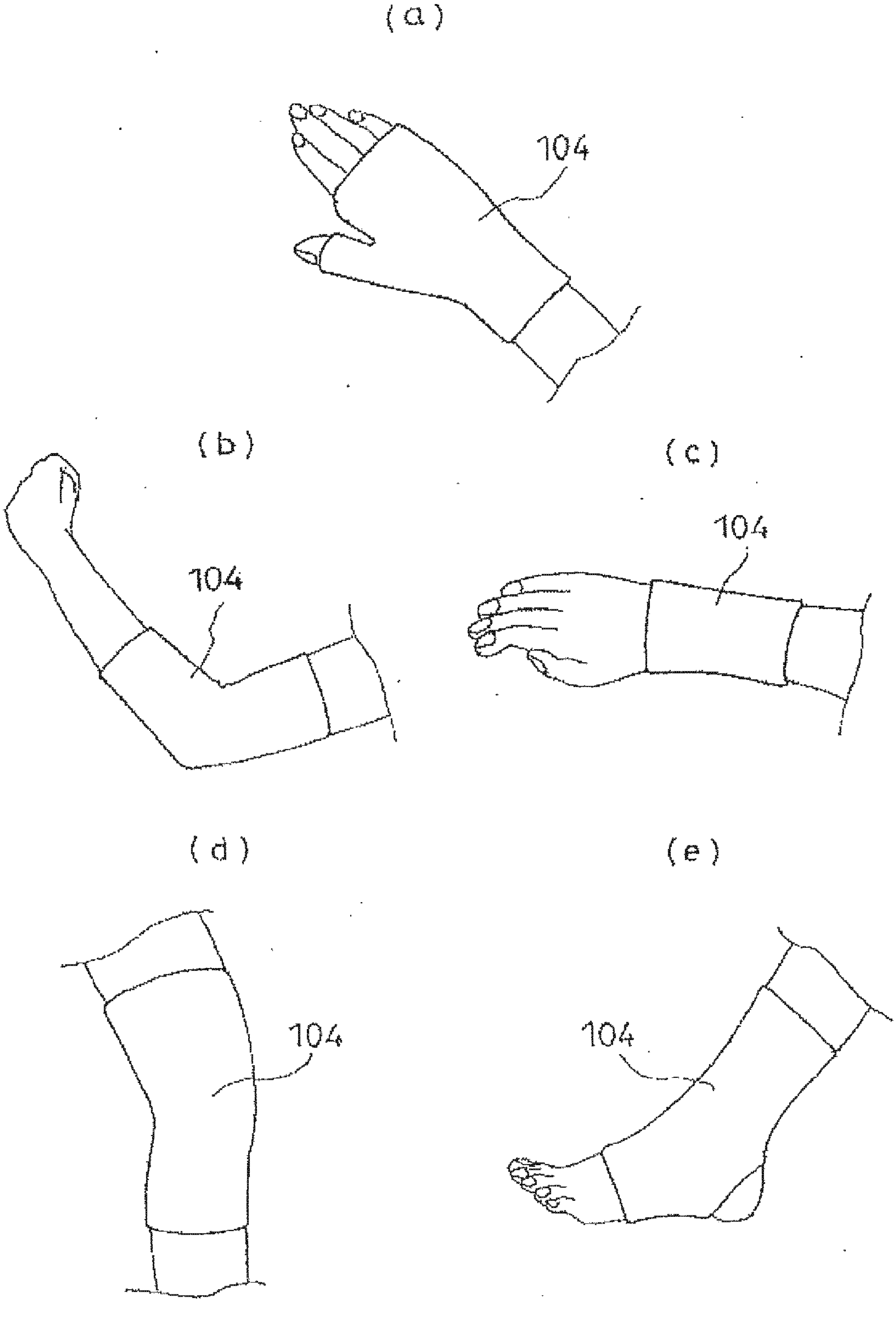

Fig.12

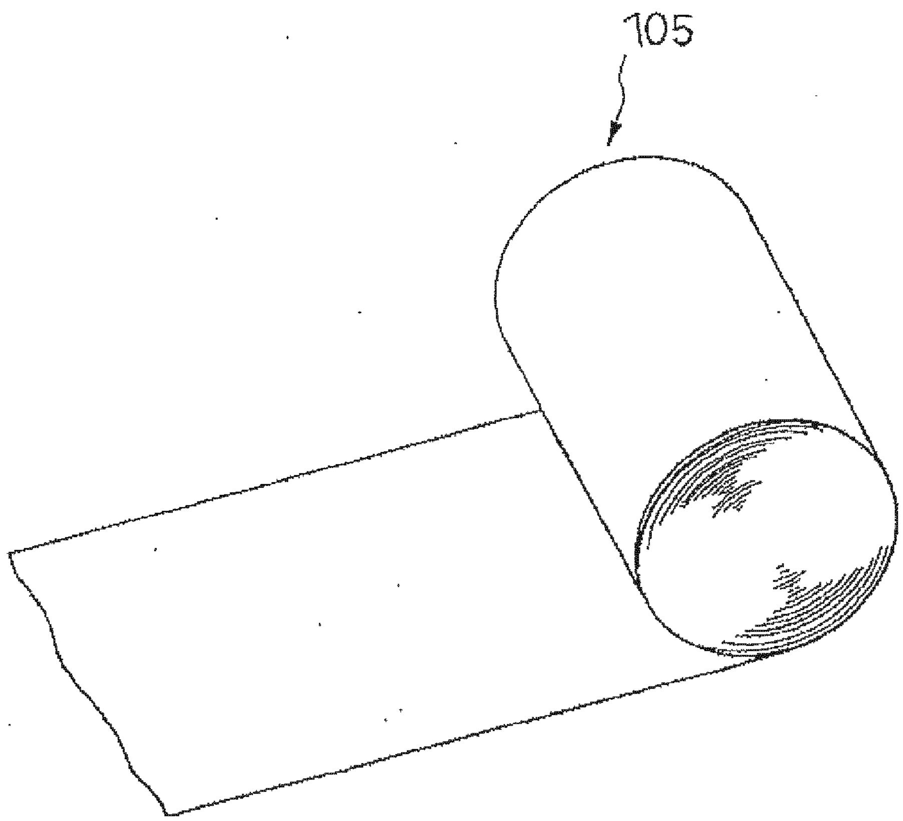

Fig.13

(a)

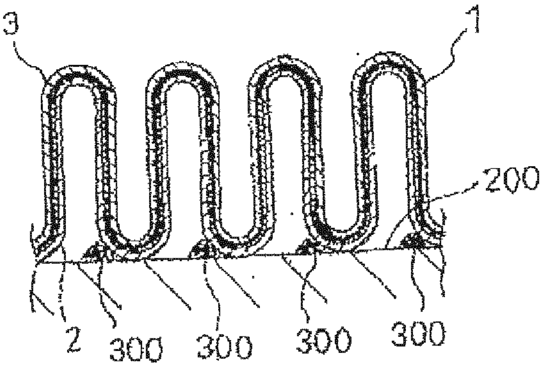


(b)

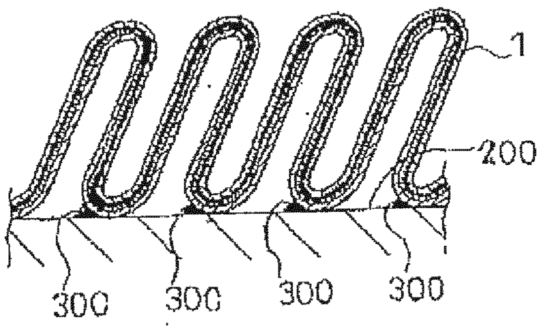


(c)

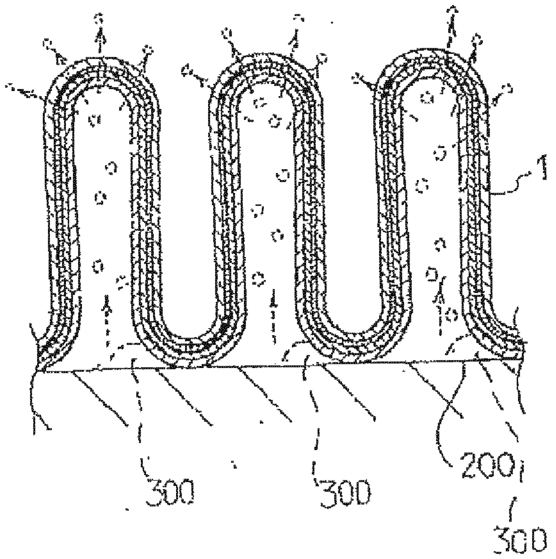

Fig.14

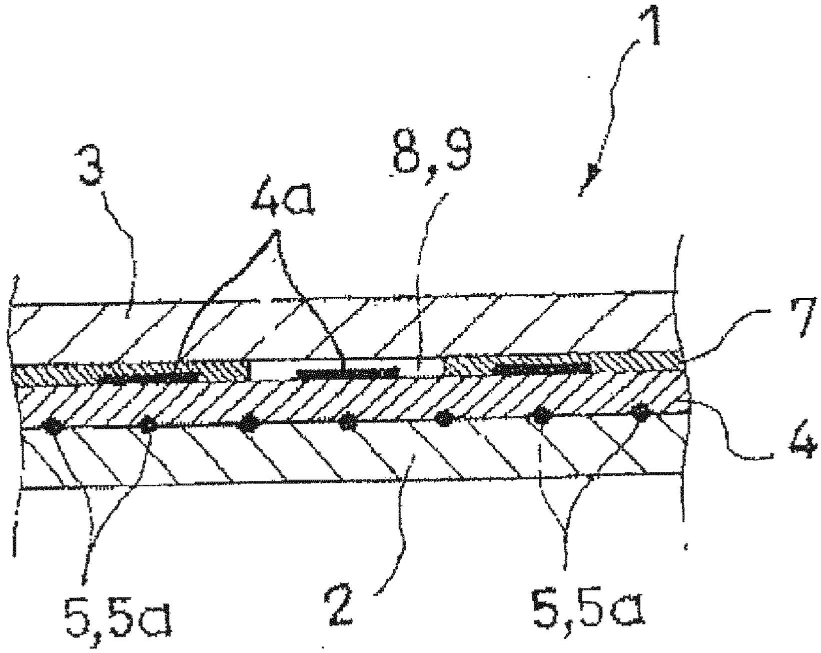

Fig.15

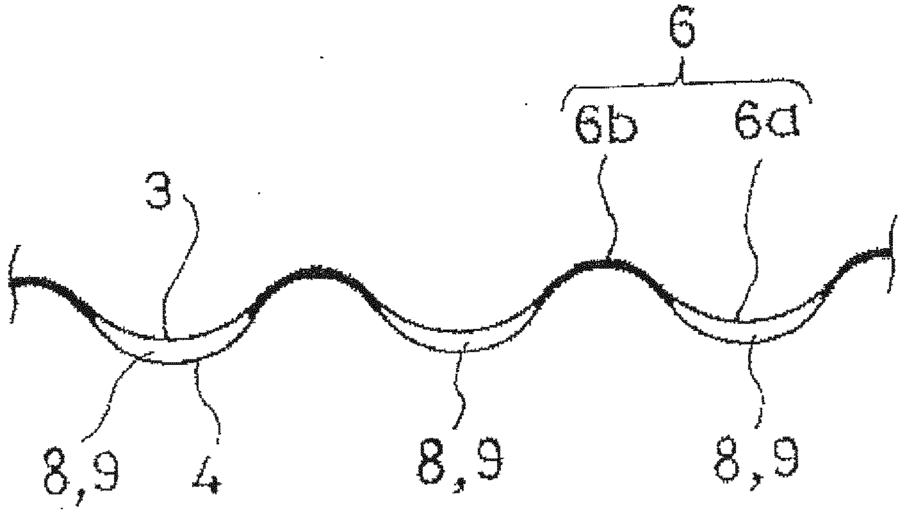

Fig.16

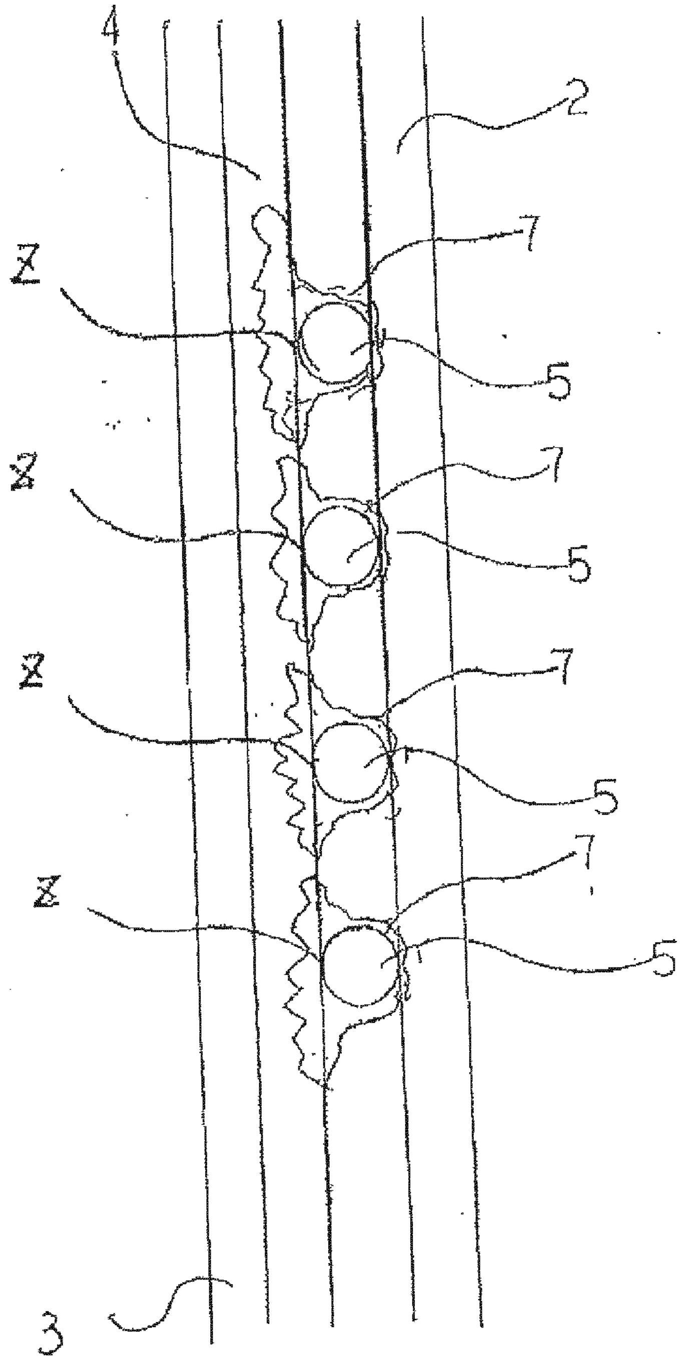

Fig.17

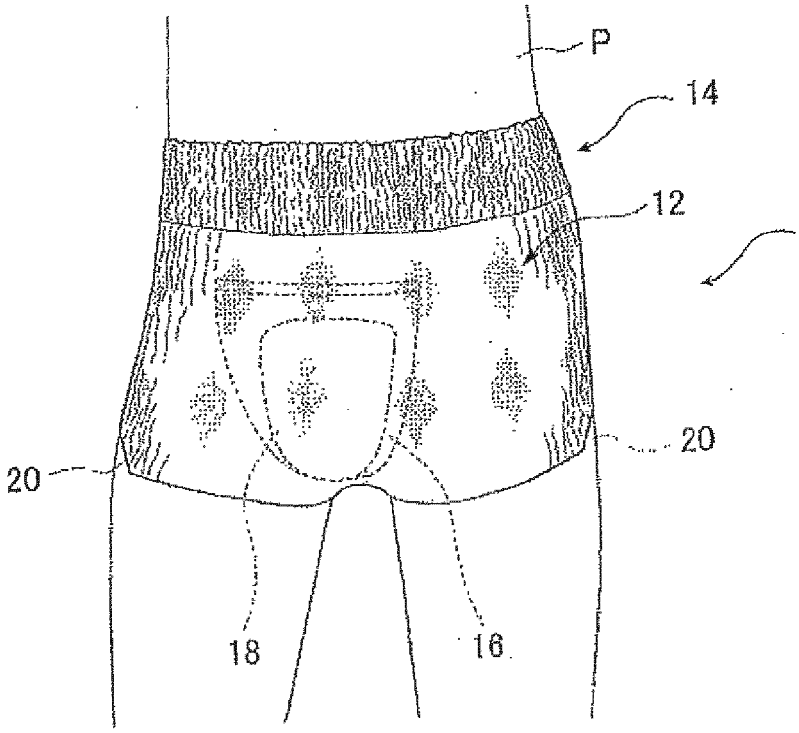

Fig.18

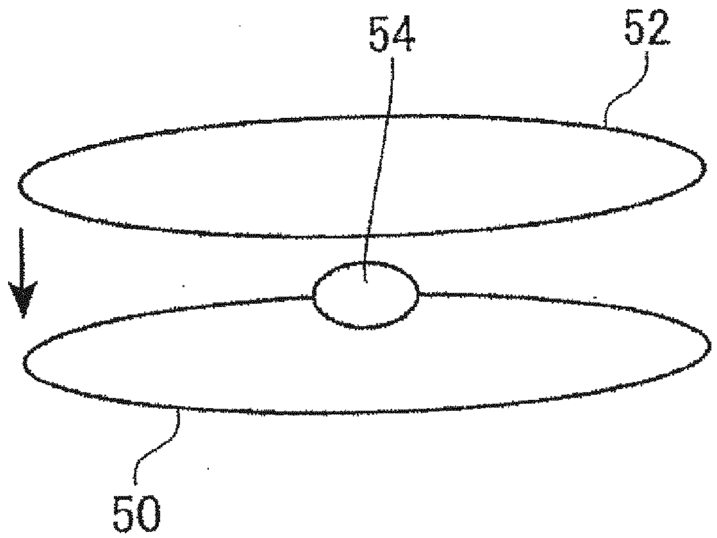

CONFIRMATION COPY

Fig.19

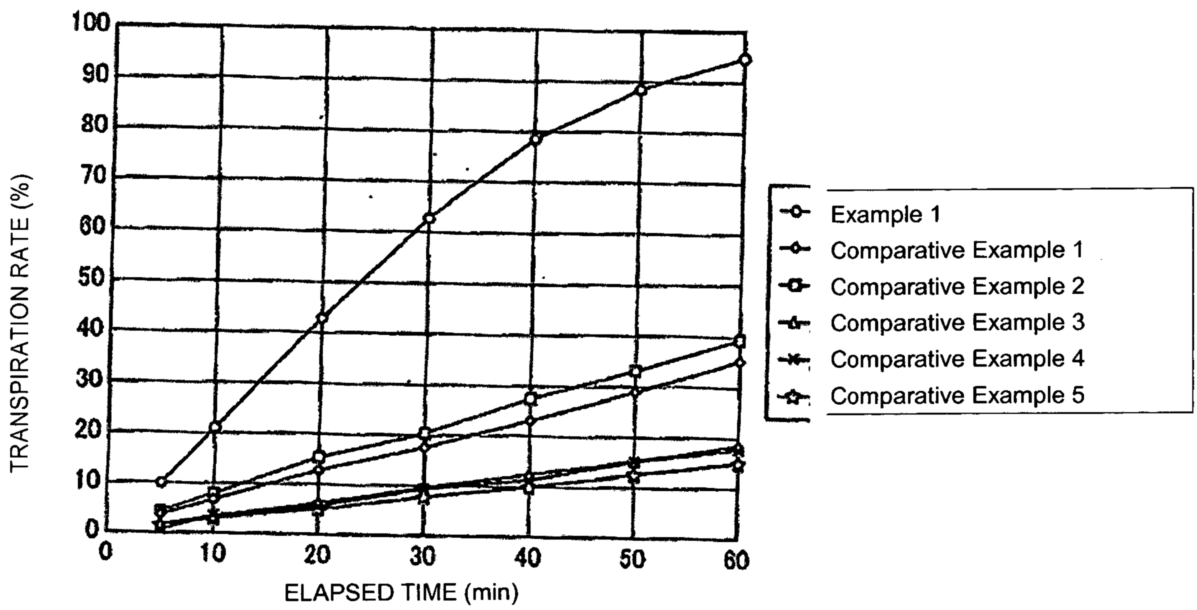

Fig.20

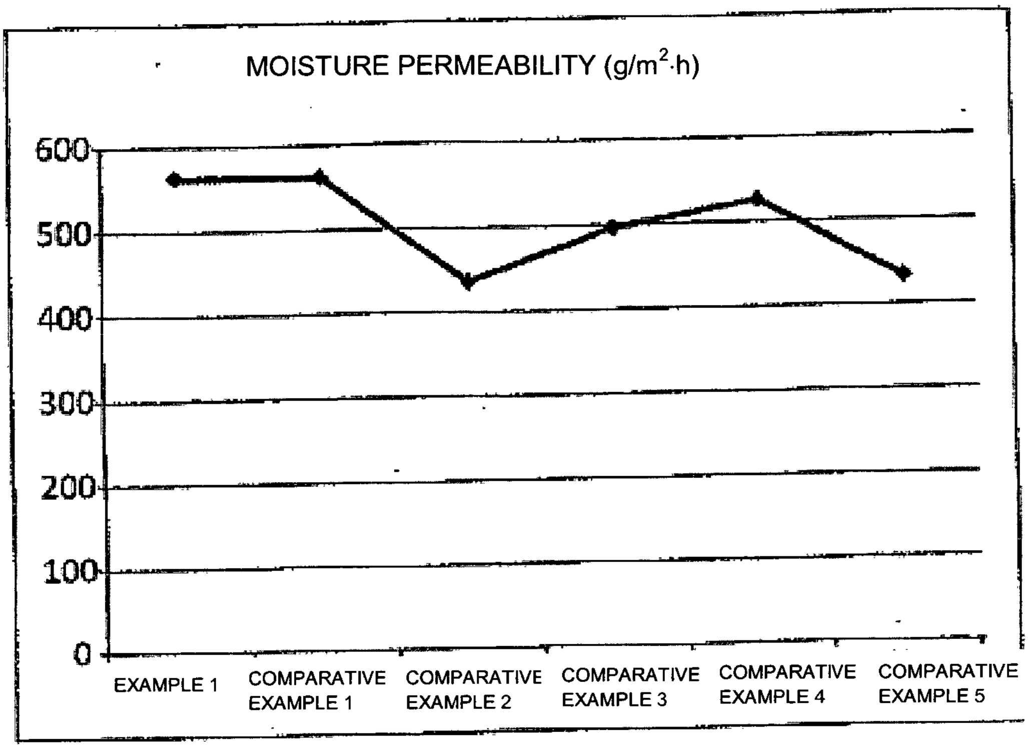
MOISTURE PERMEABILITY (g/m²·h)
600
500
400
300
200
100
0
EXAMPLE 1
COMPARATIVE EXAMPLE 1
COMPARATIVE EXAMPLE 2
COMPARATIVE EXAMPLE 3
COMPARATIVE EXAMPLE 4
COMPARATIVE EXAMPLE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3667267 B
- JP 2009097104 A
- WO 2015046401 A
- EP 0604731 A
- JP 2009297096 B
- WO 2014196669 A